# EUROPEAN PATENT APPLICATION

(11) **EP 2 301 894 A1**
(43) Date of publication of application: **30.03.2011**
(21) Application number: 09770098.3
(22) Date of filing: 19.06.2009
(51) Int. Cl.: C02F 1/46, A61L 2/02, A61L 2/18, C02F 1/461

(54) **STERILIZATION METHOD AND STERILIZATION DEVICE**

(30) Priority: 23.06.2008 JP 2008163327
(71) Applicant: Tanah Process Ltd., Osaka 545-0035 (JP)
(72) Inventor: TANAHASHI Seiji, Osaka-shi Osaka 545-0035 (JP); TANAHASHI Masakazu, Osaka-shi Osaka 545-0035 (JP)
(74) Representative: Hart-Davis, Jason
(86) International application number: PCT/JP2009/061236
(87) International publication number: WO 2009/157388

(57) **Abstract**

The disinfection method of the present invention includes steps (i) and (ii). In step (i), a voltage is applied, in an aqueous liquid (21), between a counter electrode (13) and a first ion-adsorbing electrode (11) containing a first electrically conductive material (11a) capable of adsorbing an ion reversibly, thereby changing a pH of the aqueous liquid (21) to a value less than 5 or to a value greater than 9. In step (ii), the pH of the aqueous liquid 21 is adjusted to a range of 5 to 9.

## Description

### TECHNICAL FIELD

The present invention relates to disinfection methods and disinfection devices, for example, methods and devices for disinfecting aqueous liquids. Moreover, the present invention relates to methods and devices for disinfecting objects such as instruments.

### BACKGROUND ART

Examples of methods for killing germs in water includes a method using an oxidative decomposition reaction caused by peracetic acid and a method using a reduction reaction of phtharal. Although these methods are suitable for washing medical instruments and the like, they are not suitable for the use in which the disinfected water is utilized. Moreover, these methods require chemicals, such as peracetic acid and phtharal, for disinfection.

As a disinfection device for utilizing disinfected water, a device that releases disinfectant metal ions by electrolysis has been proposed (JP 2000-153278 A). However, the proposed device is not suitable for producing drinking water, and therefore the use thereof is limited. Furthermore, a water purifier equipped with a filtration member containing an antibacterial agent has been proposed (JP H5-309370 A). In the proposed water purifier, although the increase of various bacteria in the filtration member is suppressed, the produced purified water is not adequately disinfected. Furthermore, an electrolytic cell for disinfecting drinking water with electrolysis has been also proposed (JP H7-108274 A).

### CITATION LIST

### Patent Literature

PTL 1: JP 2000-153278 A
PTL 2: JP H5-309370 A
PTL 3: JP H7-108274 A

JP H7-08274 A discloses that the electrolysis produces radical nascent oxygen, which causes disinfection (paragraph [0005] of JP H7-108274 A). However, because such radical oxygen has a short lifetime, it seems difficult fully to disinfect water that flows at a distance from the electrode only with the radical oxygen.

### SUMMARY OF INVENTION

With the foregoing background in mind, it is an object of the present invention to provide a novel disinfection method and a novel disinfection device.

In order to achieve the above-mentioned object, the present inventors diligently studied and found that an aqueous liquid can be disinfected by a specific operation using a specific electrode. The present invention is based on these new findings.

The disinfection method of the present invention includes the steps of: (i) applying a voltage, in an aqueous liquid, between a counter electrode and a first ion-adsorbing electrode containing a first electrically conductive material capable of adsorbing an ion reversibly, thereby changing a pH of the aqueous liquid to a value less than 5 or to a value greater than 9; and (ii) adjusting the pH of the aqueous liquid to a range of 5 to 9, wherein step (i) and step (ii) are performed in this order.

The disinfection device of the present invention includes a first ion-adsorbing electrode, a counter electrode, and a power supply for applying a voltage between the first ion-adsorbing electrode and the counter electrode, wherein the first ion-adsorbing electrode contains a first electrically conductive material capable of adsorbing an ion reversibly, and the disinfection device performs the steps of: (i) applying a voltage between the first ion-adsorbing electrode and the counter electrode in an aqueous liquid, thereby changing a pH of the aqueous liquid to a value less than 5 or to a value greater than 9; and (ii) adjusting the pH of the aqueous liquid to a range of 5 to 9, wherein step (i) and step (ii) are performed in this order.

The present invention can disinfect specific objects (aqueous liquids, instruments, etc.) using a simple device. The disinfection device of the present invention is easy to maintain. Moreover, the method and device of the present invention do not need special chemicals for disinfection. Because the method and device of the present invention can disinfect an aqueous liquid or an instrument with low electric power, they are useful especially in an area or situation with no electric power supply (e.g., at the time of a disaster).

### BRIEF DESCRIPTION OF DRAWINGS

Fig. 1A is an illustrative view showing a disinfection device of the present invention.
Fig. 1B shows an operation of the disinfection device shown in Fig. 1A.
Fig. 1C shows an operation of the disinfection device shown in Fig. 1A.
Fig. 2A is an illustrative view showing another disinfection device of the present invention.
Fig. 2B shows an operation of the disinfection device shown in Fig. 2A.
Fig. 2C shows an operation of the disinfection device shown in Fig. 2A.
Fig. 3A is an illustrative view showing still another disinfection device of the present invention.
Fig. 3B shows an operation of the disinfection device shown in Fig. 3A.
Fig. 3C shows an operation of the disinfection device shown in Fig. 3A.
Fig. 3D shows an operation of the disinfection device shown in Fig. 3A.
Fig. 3E is a flow chart showing an operation of the disinfection device shown in Fig. 3A.
Fig. 3F shows an operation of the disinfection device shown in Fig. 3A.
Fig. 4A is an illustrative view showing yet another disinfection device of the present invention.
Fig. 4B shows an operation of the disinfection device shown in Fig. 4A.
Fig. 4C shows an operation of the disinfection device shown in Fig. 4A.
Fig. 4D shows an operation of the disinfection device shown in Fig. 4A.
Fig. 5A is an illustrative view showing yet another disinfection device of the present invention.
Fig. 5B shows an operation of the disinfection device shown in Fig. 5A.
Fig. 5C shows the electric potential in the operation shown in Fig. 5B.
Fig. 6 is an illustrative view showing yet another disinfection device of the present invention.
Fig. 7 is an illustrative view showing yet another disinfection device of the present invention.
Fig. 8 is an illustrative view showing yet another disinfection device of the present invention.
Fig. 9 is an illustrative view showing yet another disinfection device of the invention.
Fig. 10 schematically shows a part of the disinfection device shown in Fig. 9.
Fig. 11 is a flow chart showing an operation of the disinfection device shown in Fig. 9.
Fig. 12 shows an ion-adsorbing electrode used in the disinfection device of the invention.
Fig. 13A is a top view showing the disinfection device used in Examples.
Fig. 13B is a side view showing the ion-adsorbing electrode used in Examples.
Fig. 13C is a side view showing the counter electrode used in Examples.

### DESCRIPTION OF EMBODIMENTS

Hereinafter, embodiments of the present invention are described. In the following description, embodiments of the present invention are described by way of example, but the present invention is not limited to the examples described below. In the following description, specific values and specific materials are described as examples. However, other values and materials may be used as long as the advantageous effect of the present invention can be achieved.

### [Disinfection method]

The method of the present invention is a method for disinfecting specific objects (for example, liquids, instruments, etc.). The method of the present invention can disinfect an aqueous liquid containing ions other than hydrogen ion (H+) and hydroxide ion (OH-). Hereinafter, ions other than hydrogen ion (H+) and hydroxide ion (OH-) may be referred to as "ions (L)." The method of the present invention includes the following steps (i) and (ii).

In step (i), a voltage is applied, in an aqueous liquid, between a counter electrode and a first ion-adsorbing electrode containing a first electrically conductive material capable of adsorbing an ion reversibly, thereby changing a pH of the aqueous liquid to a value less than 5 or a value greater than 9. That is, in step (i), the pH of the aqueous liquids having a pH of 5 or more (e.g., an aqueous liquid having a pH in the range of 5 to 9) is changed to a value less than 5 (acidic), or the pH of the aqueous liquid having a pH of 9 or less (e.g., an aqueous liquid having a pH in the range of 5 to 9) is changed to a value greater than 9 (alkaline). For example, the pH of an aqueous liquid having a pH in the range of 5 to 9 may be changed to a value of 4 or less (acidic). Alternatively, the pH of an aqueous liquid having a pH in the range of 5 to 9 may be changed to a value of 10 or more (alkaline). The aqueous liquid can be disinfected through step (i).

In this specification, examples of aqueous liquids having a pH less than 5 include an aqueous liquid having a pH of 4.5 or less, an aqueous liquid having a pH of 4 or less, and an aqueous liquid having a pH of 3.5 or less. Moreover, examples of aqueous liquids having a pH greater than 9 include an aqueous liquid having a pH of 9.5 or more, an aqueous liquid having a pH of 10 or more, and an aqueous liquid having a pH of 10.5 or more. Moreover, examples of aqueous liquids having a pH in the range of 5 to 9 the aqueous liquid having a pH in the range of 5.5 to 8.5 and an aqueous liquid having a pH in the range of 6 to 8.

In step (i), a voltage is applied between the first ion-adsorbing electrode and the counter electrode so that ions (L) are adsorbed on the first electrically conductive material and so that water is electrolyzed at the counter electrode. In step (i), the electrodes to which the voltage is applied are placed so as to be in contact with the aqueous liquid. For example, the electrodes to which the voltage is applied are immersed in the aqueous liquid.

Step (i) may be performed as a batch process or may be performed as a flow-through process. The batch process allows the disinfection effect to be enhanced. The flow-through process makes it possible to disinfect a large amount of the aqueous liquid. Steps other than step (i) usually are performed as a batch process but may be performed as other processes (e.g., a flow-through process).

In the case where step (i) is performed as a flow-through process, the first ion-adsorbing electrode and the counter electrode may be placed in a bath connected to a system including the aqueous liquid. Moreover, step (i) may be performed in a state where the aqueous liquid flows through the bath continuously.

The flow-through process is a process in which a liquid is introduced to and discharged from a bath continuously. Here, consideration is given to a case where the voltage is applied in the flow-through process. In the flow-through process, when a voltage is applied under conditions in which the concentration of the ions (L) differs greatly between the upstream side into which the liquid flows and the downstream side from which the liquid is discharged, the downstream side of the electrically conductive material sometimes cannot adsorb the ions sufficiently even if the upstream side of the electrically conductive material has reached its ion adsorption capacity. In that case, the entire electrically conductive material in the ion-adsorbing electrode cannot be used efficiently. Moreover, because the ions (L) are adsorbed on the upstream side of the electrically conductive material, the concentration of the ions (L) in the downstream side becomes low. Accordingly, the resistance of the liquid in the downstream becomes high and the voltage drop (IR drop) caused by the liquid becomes large in the downstream. Under such conditions, if a high voltage is applied so that water is electrolyzed at the counter electrode, water is electrolyzed in some cases at the surface of the electrically conductive material of the ion-adsorbing electrode on the upstream side where the voltage drop caused by the liquid is small. If water is electrolyzed at the surface of the electrically conductive material, the generated gases degrade the performance of the electrically conductive material. For these reasons, the flow-through process may cause these problems: (1) the entire ion-adsorbing electrode cannot be used efficiently; (2) the performance of the electrically conductive material in the ion-adsorbing electrode deteriorates. In contrast, the batch process has the advantage that it does not cause these problems. In order to avoid the above-mentioned problems, when step (i) is performed as a flow-through process, it is preferred to perform step (i) under the conditions where the concentration of the ions (L) in the liquid does not differ greatly between the upstream side into which the liquid flows and the downstream side from which the liquid is discharged.

It should be noted that the "batch process" means a process in which a liquid in a bath is treated while the liquid in the bath is not substantially replaced during a single step. Generally, when the treatment of the aqueous liquid is completed in the batch process, the aqueous liquid in the bath is discharged and another liquid is introduced in the bath. Generally, addition to or discharge of the aqueous liquid inside the bath is not carried out until the treatment is completed. The treatment is considered as a batch process as long as the liquid inside the bath is not substantially replaced until the treatment is completed. In other words, even if a small amount of the aqueous liquid that does not affect the treatment is added or discharged, the treatment is considered as a batch process. For example, even when 20 vol% or less (for example, 10 vol% or less, 5 vol% or less, or 1 vol% or less) of the aqueous liquid inside the bath is added or discharged during the treatment, it can be considered as a batch method.

The aqueous liquid is a liquid containing water and the water content thereof is, for example, greater than 50 wt%, greater than 75 wt%, or greater than 90 wt%. In a typical example, the medium of the aqueous liquid is only water. As long as the advantageous effect of the present invention can be achieved, the aqueous liquid may contain alcohols, etc. The aqueous liquid typically is an aqueous solution containing ions other than hydrogen ion (H⁺) and hydroxide ion (OH⁻). Examples of such aqueous solutions include tap water, river water, lake water, sea water, rain water, well water, spring water, ground water, etc.

The conductivity of the aqueous liquid may be in the range of 50 µS/cm to 10 mS/cm, or may be in the range of 100 µS/cm to 500 µS/cm. The disinfection method and disinfection device of the present invention are capable of changing the pH of an aqueous liquid in which the concentration of the ions (L) is relatively low. Specifically, an aqueous liquid having a conductivity of 500 µS/cm or less (for example, 100 µS/cm or less) can be used.

It should be noted that when the concentration of the ions (L) in the aqueous liquid is too low, the disinfection method and disinfection device of the present invention cannot change the pH greatly in some cases. In such cases, a salt may be added to the aqueous liquid. Although the salt to be added is not limited, it is preferred to choose the salt in view of the use of the aqueous liquid after disinfection. Examples of salts to be added include sodium nitrate, sodium chloride, calcium chloride, potassium sulfate, potassium acetate, etc. Alternatively, the concentration of the ions (L) may be controlled by releasing the ions (L) that previously have been adsorbed by the ion-adsorbing electrode into the aqueous liquid.

Step (ii) is performed after step (i). In step (ii), the pH of the aqueous liquid is adjusted to the range of 5 to 9 (for example, the range of 6 to 8). Potable water can be obtained by adjusting the pH to a range of 5 to 9 (a range of neutral or near neutral). Moreover, when instruments, etc. made of metal are to be disinfected, the corrosion of the instruments can be presented by washing them after disinfection with the aqueous liquid having a pH in the range of 5 to 9.

Examples of methods to perform the above-mentioned steps include the following methods. A series of steps performed in each of the following examples may be repeated two or more times.

### [First method]

In the disinfection method of the present invention, step (ii) may be performed by applying a voltage between the first ion-adsorbing electrode and a counter electrode in the aqueous liquid. In this case, the disinfection method includes the following two examples.

### [First example of the first method]

In step (i) in the first example, the pH of the aqueous liquid is changed to a value less than 5 (e.g., 4 or less) by applying a voltage between the first ion-adsorbing electrode and the counter electrode in the aqueous liquid so that the first ion-adsorbing electrode serves as a cathode.

By applying a voltage between the first ion-adsorbing electrode and the counter electrode so that the first ion-adsorbing electrode serves as a cathode (negative electrode) and so that the counter electrode serves as an anode (positive electrode), cations in the aqueous liquid are adsorbed on the first electrically conductive material and water is electrolyzed at the counter electrode. The water electrolysis at the counter electrode generates hydrogen ion (H⁺) and oxygen gas. Consequently, the voltage application in step (i) reduces the pH of an aqueous liquid and changes the electric potential of the aqueous liquid into a high oxidative potential. As a result, the aqueous liquid is disinfected. Moreover, the concentration of cations other than hydrogen ion in the aqueous liquid decreases by the voltage application in step (i).

In step (ii) in the first example, the pH of the aqueous liquid is adjusted so the range of 5 to 9 by applying a voltage between the first ion-adsorbing electrode and the counter electrode in the aqueous liquid so that the first ion-adsorbing electrode serves as an anode. With this voltage application, cations adsorbed on the first electrically conductive material are released into the aqueous liquid and water is electrolyzed at the counter electrode. The water electrolysis at the counter electrode generates hydroxide ion (OH⁻) and hydrogen gas. Consequently, the pH of the aqueous liquid is raised by the voltage application in step (ii). The concentration of the ions (L) in the aqueous liquid after step (ii) can be almost the same as that before starting step (i).

### [Second example of the first method]

In step (i) in the second example, the pH of the aqueous liquid is changed to a value greater than 9 (e.g., 10 or more) by applying a voltage between the first ion-adsorbing electrode and the counter electrode in the aqueous liquid so that the first ion-adsorbing electrode serves as an anode.

By applying a voltage between the first ion-adsorbing electrode and the counter electrode so that the first ion-adsorbing electrode serves as an anode (positive electrode) and so that the counter electrode serves as a cathode (negative electrode), anions in the aqueous liquid are adsorbed on the first electrically conductive material and water is electrolyzed at the counter electrode. The water electrolysis at the counter electrode generates hydroxide ion (OH⁻) and hydrogen gas. Consequently, the voltage application in step (i) raises the pH of the aqueous liquid and changes the electric potential of the aqueous liquid into a low reductive potential. As a result, the aqueous liquid is disinfected. Moreover, the concentration of anions other than hydroxide ion in the aqueous liquid is decreased by the voltage application in step (i).

In step (ii) in the second example, the pH of the aqueous liquid is adjusted to the range of 5 to 9 by applying a voltage between the first ion-adsorbing electrode and the counter electrode in the aqueous liquid so that the first ion-adsorbing electrode serves as a cathode. With this voltage application, anions adsorbed on the first electrically conductive material are released into the aqueous liquid and water is electrolyzed at the counter electrode. The water electrolysis at the counter electrode generates hydrogen ion and oxygen gas. Consequently, the pH of the aqueous liquid is decreased by the voltage application in step (ii). The concentration of the ions (L) in the aqueous liquid after step (ii) can be almost the same as that before starting step (i).

To electrolyze water at the counter electrode, it is generally necessary to apply a voltage of 2 volts or more between the electrodes. The same applies not only to step (i) but also to other steps in which water is electrolyzed at the counter electrode. When the voltage drop (IR drop) caused by the resistance of the aqueous liquid is large, applying a higher voltage is required. In an example, a voltage to be applied is in the range of 2 volts to 50 volts (for example, 2 volts to 20 volts).

### [Second method]

In the disinfection method of the present invention, step (ii) may be performed by applying a voltage between a second ion-adsorbing electrode and a counter electrode in the aqueous liquid. The second ion-adsorbing electrode contains a second electrically conductive material capable of adsorbing an ion reversibly. This disinfection method uses the first and second ion-adsorbing electrodes. This disinfection method includes the following two examples.

### [First example of the second method]

In step (i) in the first example, the pH of the aqueous liquid is changed to a value less than 5 (e.g., 4 or less) by applying a voltage between the first ion-adsorbing electrode and the counter electrode so that the first ion-adsorbing electrode serves as a cathode. Next, in step (ii), the pH of the aqueous liquid is adjusted to the range of 5 to 9 by applying a voltage between the second ion-adsorbing electrode and the counter electrode so that the second ion-adsorbing electrode serves as an anode.

### [Second example of the second method]

In step (i) in the second example, the pH of the aqueous liquid is changed to a value greater than 9 (e.g., 10 or more) by applying a voltage between the first ion-adsorbing electrode and the counter electrode so that the ion-adsorbing electrode serves as an anode. Next, in step (ii), the pH of the aqueous liquid is adjusted to the range of 5 to 9 by applying a voltage between the second ion-adsorbing electrode and the counter electrode so that the second ion-adsorbing electrode serves as a cathode.

In the second method, the ions (L) are adsorbed by the first and second ion-adsorbing electrodes after step (ii). Accordingly, the second method can reduce the concentration of the ions (L) in the aqueous liquid. When the concentration of the ions in the aqueous liquid after the disinfection treatment needs to be almost the same as that before the disinfection treatment, a voltage may be applied between the ion-adsorbing electrode and the counter electrode in the direction that is opposite to the direction in the above-mentioned step (the same applies to other methods). By applying a voltage between the first ion-adsorbing electrode and the counter electrode in the direction that is opposite to the direction in the above-mentioned step, the ions adsorbed by the first ion-adsorbing electrode can be released. By applying a voltage between the second ion-adsorbing electrode and the counter electrode in the direction that is opposite to the direction in the above-mentioned step, the ions adsorbed by the second ion-adsorbing electrode can be released. Moreover, by applying a voltage between the first ion-adsorbing electrode and the second ion-adsorbing electrode in an appropriate direction, the ions adsorbed by both of the electrodes can be released. Moreover, by short-circuiting the first ion-adsorbing electrode and the second ion-adsorbing electrode, the ions adsorbed by the both electrodes can be released.

The second method may include another step (y) before or after step (ii). In step (y), the concentration of ions in the aqueous liquid is reduced by applying a voltage between the first ion-adsorbing electrode and the second ion-adsorbing electrode. This step generally is performed after step (ii). By performing step (y), the concentration of the ions (L) can be reduced further.

### [Third method]

The disinfection method of the present invention also may contain another step (x) between step (i) and step (ii). In step (x), when the pH of the aqueous liquid that has been subjected to step (i) is less than 5, the pH of the aqueous liquid is changed to a value greater than 9, and when the pH of the aqueous liquid that has been subjected to step (i) is greater than 9, the pH of the aqueous liquid is changed to a value less than 5. In an example of step (x), the pH of the aqueous liquid that has been changed to a value not greater than 4 or not less than 10 in step (i) is changed by 6 or more to a value not greater than 4 or not less than 10. The phrase "the pH is changed by 6 or more" means that the pH of the aqueous liquid is changed to a value not less than 10 in step (x) when the pH of the aqueous liquid has been changed to a value not greater than 4 in step (i), and the pH of the aqueous liquid is changed to a value not greater than 4 in step (x) when the pH of the aqueous liquid has been changed to a value not less than 10 in step (i). Examples of disinfection methods including step (x) include the following three examples.

### [First of the third method]

In the first example, step (x) and step (ii) are performed by applying a voltage between the first ion-adsorbing electrode and the counter electrode in the aqueous liquid. That is, steps (i), (x), and (ii) are performed by applying a voltage between the first ion-adsorbing electrode and the counter electrode. The direction of the voltage applied to the counter electrode in step (i) and step (ii) is opposite to that in step (x) (the same applies to the following second and third examples). In this example, the ions adsorbed on the electrically conductive material are released into the aqueous liquid in step (ii). Consequently, the concentration of the ions (L) in the aqueous liquid after step (ii) is almost the same as the concentration of the ions (L) in the aqueous liquid before starting step (i).

After step (ii), the first example of the third method may include step (y) of reducing the concentration of the ions in the aqueous liquid by applying a voltage between the first ion-adsorbing electrode and a second ion-adsorbing electrode in the aqueous liquid. The second ion-adsorbing electrode contains a second electrically conductive material capable of adsorbing an ion reversibly.

### [Second example of the third method]

In the second example of the third method, step (x) is performed by applying a voltage between a second ion-adsorbing electrode and a counter electrode in the aqueous liquid. The second ion-adsorbing electrode contains a second electrically conductive material capable of adsorbing an ion reversibly. Next, step (ii) is performed by applying a voltage between the first ion-adsorbing electrode and the counter electrode in the aqueous liquid. In this case, the concentration of the ions (L) in the aqueous liquid after step (ii) is lower than that before starting step (i).

### [Third example of the third method]

In the third example of the third method, step (x) is performed by applying a voltage between the first ion-adsorbing electrode and the counter electrode in the aqueous liquid. Moreover, step (ii) is performed by applying a voltage between a second ion-adsorbing electrode and a counter electrode in the aqueous liquid. The second ion-adsorbing electrode contains a second electrically conductive material capable of adsorbing an ion reversibly. In this case, the concentration of the ions (L) in the aqueous liquid after step (ii) is lower than that before starting step (i).

### [Fourth method]

In the disinfection method of the present invention, the above-mentioned aqueous liquid may be a first aqueous liquid, and the above-mentioned counter electrode may be a first counter electrode. Moreover, step (i) may include the following steps (i-a) and (i-b).

In step (i-a), the first ion-adsorbing electrode and the first counter electrode are immersed in the first aqueous liquid placed in a first bath. Next, the pH of the first aqueous liquid is changed to a value less than 5 (e.g., 4 or less) by applying a voltage between the first ion-adsorbing electrode and the first counter electrode. The voltage is applied so that the first ion-adsorbing electrode serves as a cathode.

In step (i-b), a second counter electrode and a second ion-adsorbing electrode containing a second electrically conductive material capable of adsorbing an ion reversibly are immersed in a second aqueous liquid placed in a second bath. Next, the pH of the second aqueous liquid is changed to a value greater than 9 (e.g., 10 or more) by applying a voltage between the second ion-adsorbing electrode and the second counter electrode. The voltage is applied so that the second ion-adsorbing electrode serves as an anode.

Either step (i-a) or step (i-b) may be performed first, or they may be performed simultaneously. The fourth method can be performed by dividing the aqueous liquid into the first aqueous liquid and the second aqueous liquid and then treating each of the aqueous liquids.

A step performed after step (i) in the fourth method may be the same step as the step performed after step (i) in the above-mentioned method. For example, step (ii) may be performed by applying a voltage between the ion-adsorbing electrode and the counter electrode in the opposite direction to the direction in step (i). The pH of the first aqueous liquid may be changed in order of {less than 5 (e.g., 4 or less)} → {greater than 9 (e.g., 10 or more)} → {5 to 9} while the pH of the second aqueous liquid may be changed in order of {greater than 9 (e.g., 10 or more)} → {less than 5 (e.g., 4 or less)} → {5 to 9}. Such a change in the pH can be achieved by controlling the direction of the voltage application and the time period of the voltage application.

### [Fifth method]

In the disinfection method of the present invention, the above-mentioned aqueous liquid may be a first aqueous liquid. Moreover, step (i) may include the following steps. The first ion-adsorbing electrode and the counter electrode are brought into contact with the first aqueous liquid placed in a first bath. The counter electrode and a second ion-adsorbing electrode containing a second electrically conductive material capable of adsorbing an ion reversibly are brought into contact with a second aqueous liquid placed in a second bath. The counter electrode is kept in an electrically floating state. In this condition, a voltage is applied between the first ion-adsorbing electrode and the second ion-adsorbing electrode, thereby changing the pH of the first aqueous liquid to a value less than 5 (e.g., 4 or less) and changing the pH of the second aqueous liquid to a value greater than 9 (e.g., 10 or more).

A performed, after step (i) in the fifth method may be the same step as the step performed after step (i) in the above-mentioned method.

In the fifth method, the counter electrode may function as a partition that divides one bath into the first bath and the second bath. This partition (i.e., the counter electrode) does not allow the aqueous liquid and ions to pass therethrough.

In the fourth and the fifth methods, step (ii) may be a step in which the first aqueous liquid and the second aqueous liquid are mixed. An almost neutral aqueous liquid can be produced by mixing the first aqueous liquid having a pH less than 5 (e.g., 4 or less) and the second aqueous liquid having a pH greater than 9 (e.g., 10 or more).

In the disinfection method of the present invention, an object (instruments, etc.) may be disinfected by immersing the object in the aqueous liquid while the voltage is applied in step (i). In a preferred example, the disinfected object is immersed in the aqueous liquid until step (ii) is completed. In the case where an object (e.g., instruments) is to be disinfected, it is more preferred to perform at least a step of changing the pH of the aqueous liquid to a value less than 5 (e.g., 4 or less). When a step of changing the pH of the aqueous liquid to a value less than 5 (e.g., 4 or less) and a step of changing the pH of the aqueous liquid to a value greater than 9 (e.g., 10 or more) are both performed, stronger disinfection can be achieved since the disinfection is performed through different conditions. In the disinfection method of the present invention, an object may be disinfected by bringing the object into contact with the aqueous liquid produced in step (i).

When disinfecting an electrically conductive object (e.g., instruments) with the above-mentioned step, a voltage may be applied between the ion-adsorbing electrode and the counter electrode with the object being immersed in the aqueous liquid and being in contact with the counter electrode. Such a method makes it easier to disinfect the object as described later because the electric potential of the object to be disinfected approaches the electric potential of the counter electrode. In this method, it is preferred for the counter electrode to have a shape that makes it easier to bring the object to be disinfected into contact with the counter electrode. For example, the counter electrode may be a basket-shaped one in which the object to be disinfected is placed. Alternatively, the counter electrode may have a hook-like portion on which the object is hung.

In the method of the present invention, the pH of the aqueous liquid may be changed to a value of 2.5 or less in the step of changing the pH of the aqueous liquid to a value less than 5. By changing the pH to a value of 2.5 or less, stronger disinfection can be achieved. In the method of the present invention, the pH of the aqueous liquid may be changed to a value of 11.5 or more in the step of changing the pH of the aqueous liquid to a value greater than 9. By changing the pH to a value of 11.5 or more, stronger disinfection can be achieved.

### [First and second ion-adsorbing electrodes]

The first and second ion-adsorbing electrodes each may include a collector that supports the first and second electrically conductive materials, and may include a collector attached on the first and second electrically conductive materials.

The first and second electrically conductive materials are each a material capable of adsorbing/releasing ions reversibly. The electrically conductive materials can be materials with a large specific surface area. In a preferred example, the electrically conductive materials include carbon materials, such as activated carbon and graphite. The electrically conductive materials may be electrically conductive sheets formed by aggregating granular activated carbon. Alternatively, the electrically conductive materials may be electrically conductive sheets formed by aggregating granular activated carbon and electrically conductive carbon. Alternatively, the electrically conductive materials may be activated carbon blocks formed by compressing activated carbon particles. Alternatively, the electrically conductive materials may be activated carbon fiber cloths, i.e. cloths formed of activated carbon fibers. The activated carbon fiber cloths that may be used herein are, for example, ACC5092-10, ACC5092-15, ACC5092-20, and ACC-5092-25 manufactured by Nippon Kynol Inc. The first electrically conductive material and the electrically conductive material may be formed of the same material, or may be formed of different materials.

The specific surface area of the electrically conductive materials is, for example, greater than 300 m²/g, and preferably is greater than 900 m²/g. The upper limit of the specific surface area is not particularly limited and, for example, may be 3000 m²/g or less, or may be 2500 m²/g or less. In this specification, the term "specific surface area" of the first and second electrically conductive materials meons a value measured with the BET method using nitrogen gas.

### [Counter electrode]

An example of the counter electrode is a metal electrode. A preferred example of the counter electrode is an electrode having a surface including a metal that allows water to be electrolyzed easily (e.g., platinum). For instance, an electrode made of titanium, an electrode made of platinum, and an electrode that is made of a metal (e.g., titanium, niobium, or tantalum) and that is coated with platinum can be used as the counter electrode. When a counter electrode is used also in a step other than step (i), the counter electrode used in step (i) and the counter electrode used in the other step may be the same counter electrode, or may be different counter electrodes. As the counter electrode, a metal sheet may be used, a metal wire may be used, or connected metal wires may be used.

Unlike the first and electrically conductive material, the surface area of the counter electrode does not need to be large. In an example, the surface area per of the counter electrode may be 100 m² or less, and may be in the range of 5×10⁻⁵ to 50 m².

### [Disinfection device]

The disinfection device of the present invention is a device for carrying out the above-mentioned disinfection method of the present invention. The description given above regarding the disinfection method can be applied to the disinfection device of the present invention, and therefore the overlapping description may be omitted. Moreover, the description regarding the disinfection device of the present invention can be applied to the disinfection method of the present invention.

The disinfection device of the present invention includes a first ion-adsorbing electrode, a counter electrode, and a power supply for applying a voltage between the first ion-adsorbing electrode and the counter electrode. The ion-adsorbing electrode contains a first electrically conductive material capable of adsorbing an ion reversibly. The disinfection device of the present invention may include a bath in which an aqueous liquid, the first ion-adsorbing electrode, and the counter electrode are placed. It should be noted that the disinfection device of the present invention may be a type where the electrodes (including the first ion-adsorbing electrode and the counter electrode) are put into the aqueous liquid. In this case, the disinfection device need not include the bath. The disinfection device of the present invention may include a pH sensor (pH meter) for monitoring the pH of the aqueous liquid. The pH of the aqueous liquid can be monitored with the pH sensor. In the case where the pH value and quantity of the aqueous liquid to be treated can be known in advance, the relationship between the conditions of voltage application (e.g., voltage application time and the amount of electric charge that flows between the electrodes) and the change in pH can be obtained previously, which allows the disinfection method of the present invention to be carried out without using the pH sensor.

The disinfection device of the present invention may include the second ion-adsorbing electrode and the second counter electrode mentioned above. Moreover, the disinfection device of the present invention may include a counter electrode that functions as a partition.

The disinfection device of the present invention carries out the disinfection method of the present invention mentioned above. Specifically, the above-mentioned steps (i) and (ii) are performed in this order. Step (i) is performed as a batch process or as a flow-through process. In addition to steps (i) and (ii), other steps mentioned above may be performed.

The disinfection device of the present invention can disinfect aqueous liquids. Moreover, the disinfection device of the present invention can disinfect objects (instruments, etc.) immersed in the aqueous liquid. In the disinfection device of the present invention, an object to be disinfected may be disinfected by immersing the object in the aqueous liquid while the voltage is applied in step (i). Moreover, an object to be disinfected may be disinfected by providing the bath, in which the object is placed, with the aqueous liquid that has been prepared in step (i) to have a pH of not greater than 5 or not less than 9. In any case, it is only necessary that the object to be disinfected is brought into contact with the aqueous liquid that has been prepared to have a pH of not greater than 5 or not less than 9.

In a preferred example to disinfect a certain object (instruments, etc.), the object is immersed in the aqueous liquid until step (ii) is completed. When disinfecting an object (e.g., instruments), it is more preferred to perform at least a step of changing the pH of the aqueous liquid to a value less than 5. When a step of changing the pH of the aqueous liquid to a value less than 5 and a step of changing the pH of the aqueous liquid to a value greater than 9 are both performed, stronger disinfection can be achieved since the disinfection is performed through different conditions.

The bath is not particularly limited as long as it can hold the aqueous liquid stably. Baths made of resins, which have a resistance for the change in pH, preferably are used, because the pH of the aqueous liquid changes. The power supply is a power supply that supplies direct current voltage. The power supply may be an AC/DC converter that converts alternating current voltage supplied from an electrical outlet into direct current voltage. Moreover, the power supply may be a primary battery such as a dry cell, or may be a secondary battery such as a lead storage battery, a nickel-metal hydride battery, and a lithium ion battery. Moreover, the power supply may be an electric generator, such as a solar cell, a wind turbine generator, and a hand driven generator. By using an electric generator as the power supply, the device of the present invention can be used in the area or the situation where electric power is not available. Such use is useful for producing drinking water in a remote place or in an emergency, etc.

Although the voltage application can be controlled manually, the disinfection device of the present invention may include a controller for performing the steps. The controller includes a processing unit (which may include an internal memory), and further include an external memory if needed. The program for performing the steps is stored in the memory. Examples of controllers include a large-scale integrated circuit (LSI). The controller is connected to various devices (power supplies, pumps, valves, etc.) and measuring instruments (e.g., pH sensors, ion concentration meters, and conductometers). The controller controls the various devices and performs the steps in accordance with the outputs from the measuring instruments.

Moreover, the disinfection device of the present invention may include an input device for inputting a target pH value or inputting a treatment method into the controller, and may include a display device for displaying the status of the treatment. Moreover, the disinfection device of the present invention may include an instrument for adding salt to the aqueous liquid when the ion concentration of the aqueous liquid is low.

In order to determine the voltage to be applied to the electrodes, the disinfection device of the present invention may include a conductometer for measuring the conductivity of the aqueous liquid, and may include a device for monitoring the gas generation at the counter electrode (e.g., a set of a light emitting device, such as an LED and a laser diode, and a photodetector, such as a photodiode). Moreover, the disinfection device of the present invention may include a voltmeter for measuring the voltage applied between the electrodes, and may include an ammeter for measuring the electric current that flows between the electrodes.

The disinfection device of the present invention may include various filters, such as hollow fiber filters and activated carbon filters. Moreover, the disinfection device of the present invention may include a device for carrying out a disinfection method other than the disinfection method of the present invention. When carrying out a plurality of disinfection methods, more reliable disinfection can be achieved.

If needed, the disinfection device of the present invention may includes a separating membrane (e.g., ion exchange membrane) that allows selected ions to pass therethrough. However, such a separating membrane generally is not needed.

When a certain object (instruments, etc.) is to be disinfected using the disinfection device of the present invention, the object to be disinfected may be brought into contact with an aqueous liquid that has been prepared in a step other than step (i), in addition to the disinfection with the aqueous liquid that has been prepared in step (i) to have a pH less then 5. The corrosion of the object can be prevented by bringing the object into contact with the aqueous liquid that has been prepared in step (ii) to have a pH of 5 to 9.

In the method and device of the present invention, more than one first ion-adsorbing electrode may be used, more than one second ion-adsorbing electrode may be used, and more than one counter electrode be used. When using a plurality of the ion-adsorbing electrodes, the function of some ion-adsorbing electrodes may be changed at every step. For example, all of the ion-adsorbing electrodes may be used as the first ion-adsorbing electrode in the first step, In a subsequent step, ion-adsorbing electrodes may be used as the first ion-adsorbing electrodes while the ion-adsorbing electrodes are used as the second ion-adsorbing electrodes.

The disinfection device of the present invention may be connected to a system including an aqueous liquid. In this case, the internal volume of the bath in which the ion-adsorbing electrode and the counter electrode are placed may be smaller than the volume of the aqueous liquid included in the system. For example, the internal volume of the bath may be 1/5 or less of the volume of the aqueous liquid included in the system. Such an embodiment makes it possible to disinfect a large amount of the aqueous liquid with a small device. When the disinfection device is connected to the system including the aqueous liquid, step (i) and other steps each independently may be performed as a batch process, or may be performed as a flow-through process. The flow-through process has the advantage that control is easy and the aqueous liquid can be treated continuously.

Moreover, in an embodiment of the disinfection device of the present invention, a plurality of the above-mentioned disinfection devices of the present invention may be connected in series or in parallel. In the case where a plurality of the disinfection devices are connected in parallel, some disinfection devices may perform a disinfection treatment in which the aqueous liquid eventually become acidic and other disinfection devices may perform a disinfection treatment in which the aqueous liquid eventually become alkaline. Moreover, step (ii) may be performed by mixing the resultant acid aqueous liquid and the resultant alkaline aqueous liquid.

Hereinafter, embodiments of the present invention will be described with reference to the drawings. In the description referring to drawings, the same reference numerals are used to indicate like parts, and a repetitive description is omitted in some cases. The drawings discussed in the following description are schematic drawings. In the drawings mentioned below, the chemical equivalent ratio is not taken into consideration for ease of understanding. In the drawings mentioned below, cations other than hydrogen ion are represented as M⁺ and anions other than hydroxide ion are represented as A-; however, the cations and anions in the aqueous liquid are not limited to univalent ions and are not limited to one species of ion. Moreover, hatching of the aqueous liquid 21 may be omitted in the drawings mentioned below. Moreover, in the drawings mentioned below, more than one ion-adsorbing electrode 11, more than one ion-adsorbing electrode 12, and more than one counter electrode 13 may be used independently. Moreover, more than one device mentioned below may be connected in series or in parallel.

### [Embodiment 1]

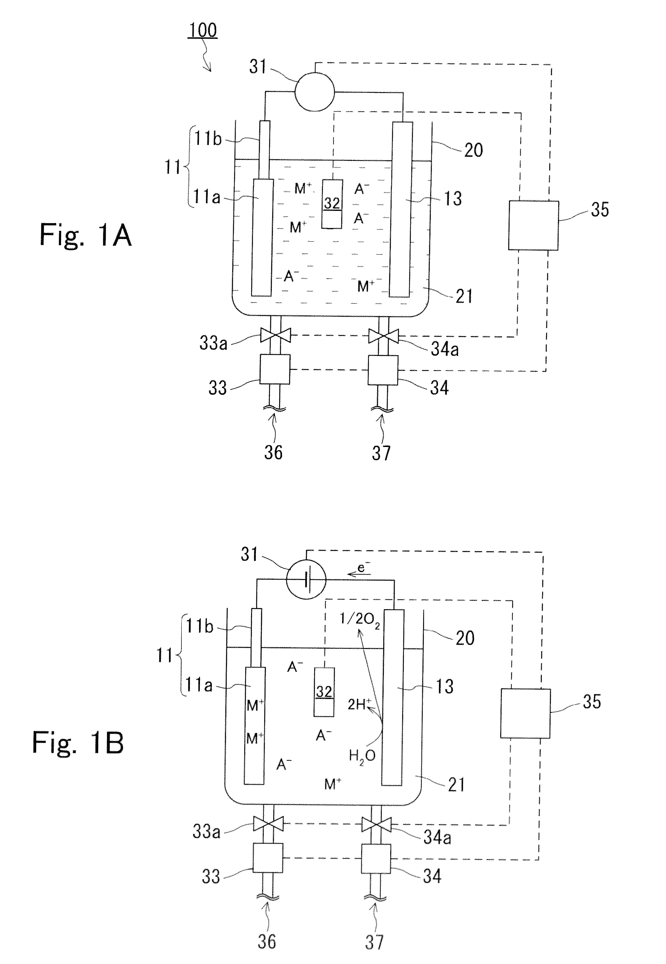

In Embodiment 1, an example is described with regard to the first example of the above-mentioned first method and a device for performing the method. Fig. 1A shows a disinfection device of Embodiment 1.

A disinfection device 100 in Fig. 1A includes an ion-adsorbing electrode (first ion-adsorbing electrode) 11, a counter electrode 13, a bath 20, a power supply 31, a pH sensor (pH meter) 32, valves 33a and 34a, pumps 33 and 34, and a controller 35. The ion-adsorbing electrode 11 includes an electrically conductive material 11a and a collector 11b. The power supply 31, the valve 33a, the valve 34a, the pump 33, and the pump 34 are controlled by the controller 35. Signals from the pH sensor 32 are inputted into the controller 35.

First, the valve 33a and the pump 33 are operated, so that the aqueous liquid 21 is introduced to the bath 20 from the inlet 36 as shown in Fig. 1A. Next, as shown in Fig. 1B, a voltage is applied between the ion-adsorbing electrode 11 and the counter electrode 13 so that the ion-adsorbing electrode 11 serves as a cathode. With this voltage application, the cation M+ in the aqueous liquid 2 is adsorbed on the electrically conductive material 11a of the ion-adsorbing electrode 11. Moreover, at the surface of the counter electrode 13, hydrogen ion and oxygen gas are generated due to the water electrolysis. As a result, the pH of the aqueous liquid 21 decreases. The voltage application is performed until the pH of the aqueous liquid 21 reaches a specific value less than 5 (e.g., 4 or less).

The method and device of Embodiment 1 provide disinfection with the acid aqueous liquid 21, disinfection with the oxidative power of the generated oxygen, and disinfection with the oxidative power of the surface of the counter electrode 13.

When the pH is 4 and the oxygen partial pressure is 1 atm, the oxidation potential E₀ = 1.228 - 0.0591pH + 0.0147logP(O₂) = 0.99 volts. When the pH is 2 and the oxygen partial pressure is 1 atm, the oxidation potential E₀ = 1.11 volts. Because the electrode potential of the counter electrode 13 is polarized due to the oxygen gas generation, the electrode potential of the counter electrode 13 is higher than the above-mentioned oxidation potential. Accordingly, strong oxidative powder works at the surface of the counter electrode 13, and thus disinfection occurs at the surface of the counter electrode 13, and the aqueous liquid 21 itself is in a state having strong oxidative power.

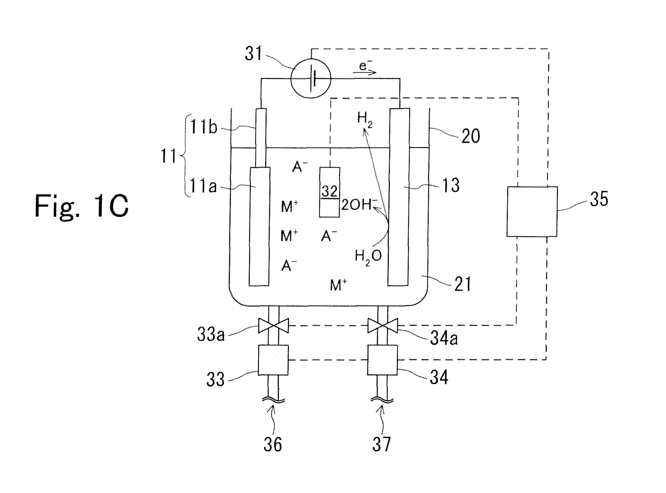

When the controller 35 detects that the pH reaches a specific value, the following step is performed immediately or after a while. Specifically, as shown in Fig. 1C, a voltage is applied between the ion-adsorbing electrode 11 and the counter electrode 13 so that the ion-adsorbing electrode 11 serves as an anode. With this voltage application, cation M+ adsorbed on the electrically conductive material 11a is released into the aqueous liquid 21. Water is electrolyzed at the counter electrode 13, and thus hydroxide ion and hydrogen gas are generated. This voltage application is performed until the pH of the aqueous liquid 21 reaches a specific value in the range of 5 to 9.

Thus the disinfection treatment of the aqueous liquid 21 is completed, and then the valve 34a and the pump 34 are operated to discharge the aqueous liquid 21, which is used as a disinfected liquid, from the outlet 37.

### [Embodiment 2]

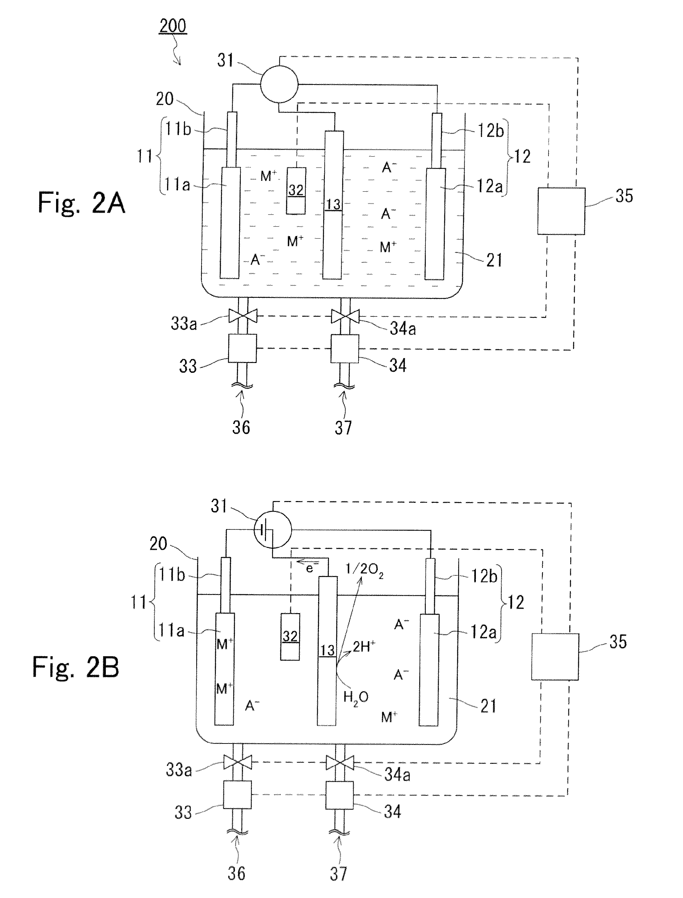

In Embodiment 2, an example is described with regard to the first example of the second method and a device for performing the method. Fig. 2A shows a disinfection device of Embodiment 2.

A disinfection device 200 in Fig. 2A includes the first ion-adsorbing electrode 11, a second ion-adsorbing electrode 12, the counter electrode 13, the bath 20, the power supply 31, the pH sensor 32, the valves 33a and 34a, the pumps 33 and 34, and the controller 35. The ion-adsorbing electrode 12 includes an electrically conductive material 12a and a collector 12b.

First, the valve 33a and the pump 33 are operated, so that the aqueous liquid 21 is introduced to the bath 20 from the inlet 36 as shown in Fig. 2A. Next, as shown in Fig. 2B, a voltage is applied between the ion-adsorbing electrode 11 and the counter electrode 13 so that the ion-adsorbing electrode 11 serves as a cathode. This step is the same as the step shown in Fig. 1B. The aqueous liquid 21 is disinfected in this step as described in Embodiment 1.

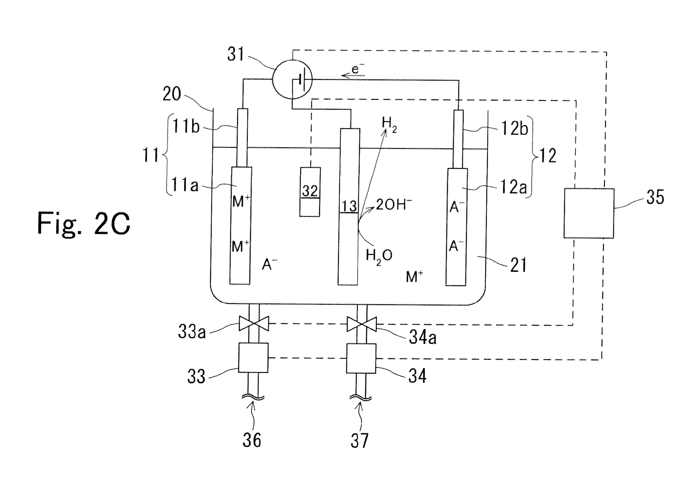

When the controller 35 detects that the pH reaches a specific value less than 5 (e.g., 4 or less), the following step is performed immediately or after a while. Specifically, as shown in Fig. 2C, a voltage is applied between the ion-adsorbing electrode 12 and the counter electrodes 13 so that the ion-adsorbing electrode 12 serves as an anode. With this voltage application, the anion A- in the aqueous liquid 21 is adsorbed by the ion-adsorbing electrodes 12. Water is electrolyzed at the counter electrode 13, and thus hydroxide ion and hydrogen gas are generated. This voltage application is performed until the pH of the aqueous liquid 21 teaches a specific value in the range of 5 to 9.

Thus the disinfection treatment of the aqueous liquid 21 is completed, and then the valve 34a and the pump 34 are operated to discharge the aqueous liquid 21, which is used as a disinfected liquid, from the outlet 37.

In the method of Embodiment 2, unless a voltage is applied between the ion-adsorbing electrode and the counter electrode in the opposite direction to that in the above-mentioned step, the ions adsorbed by the ion-adsorbing electrodes 11 and 12 are hardly released to the aqueous liquid 21. The same applies to other embodiments in which the ion-adsorbing electrode is used. Although it is not clear about this reason, it is possible, for example, that ions are attracted to the surface charge of the electrically conductive material to form an electric double layer. In the field of electric double layer capacitors, it is well known that such a phenomenon occurs. Accordingly, when the aqueous liquid 21 contains a harmful ion (e.g., heavy metal ions), the method of Embodiment 2 can reduce the concentration of the harmful ion in the aqueous liquid 21.

It should be noted that, in order to prevent excessive adsorption of ions on the electrically conductive material, it, is preferred to change the ion-adsorbing electrode periodically or to regenerate the ion-adsorbing electrode periodically. The ion-adsorbing electrode can be regenerated by releasing the ion adsorbed on the electrically conductive material. For example, when the cation M+ adsorbed on the electrically conductive material 11a is to be released, a washing aqueous liquid is introduced to the bath 20, and then a voltage is applied between the ion-adsorbing electrodes 11 and the counter electrode 13 so that the ion-adsorbing electrode 11 serves as an anode. With this voltage application, cation M+ adsorbed on the electrically conductive material 11a can be released into the washing aqueous liquid. Similarly, the anion A- adsorbed on the electrically conductive material 12a can be released into the washing aqueous liquid by applying a voltage between the ion-adsorbing electrode 12 and the counter electrode 13 so that the ion-adsorbing electrode 12 serves as a cathode. Alternatively, a voltage may be applied between the ion-adsorbing electrode 11 and the ion-adsorbing electrode 12 so that the ion-adsorbing electrode 11 serves as an anode. Alternatively, the ion-adsorbing electrode 11 and the ion-adsorbing electrode 12 may be short-circuited. When the concentration of the ions in the aqueous liquid after disinfection is desired to be almost the same as that before disinfection, the ions adsorbed on the electrically conductive material may be released with the above-mentioned method.

### [Embodiment 3]

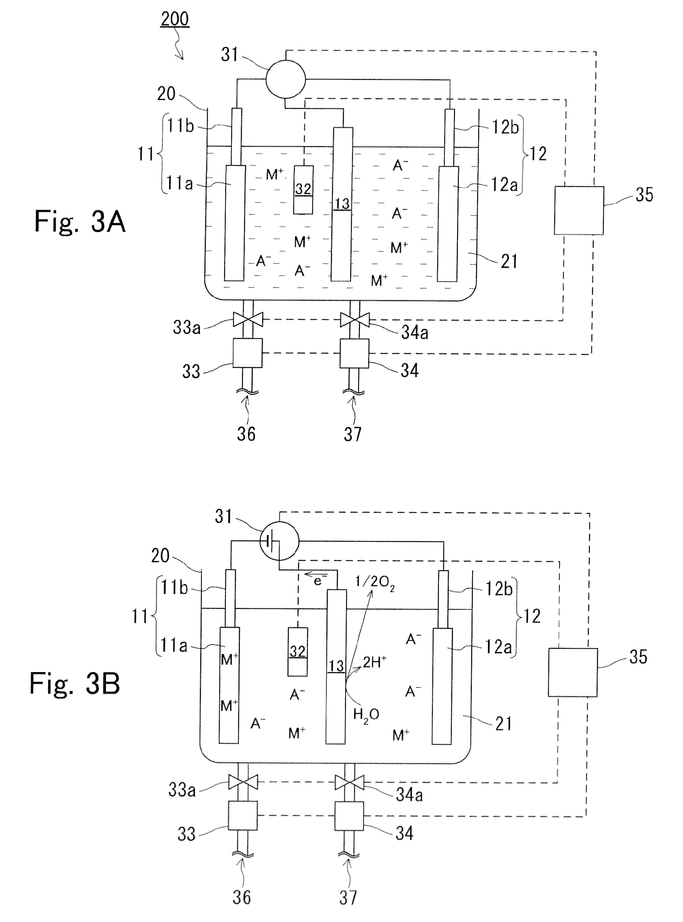

In Embodiment 3, an example is described with regard to the second example of the third method and a device for performing the method. Fig. 3A shows a disinfection device of Embodiment 3. The disinfection device 200 shown in Fig. 3A has the same structure as the device shown in Fig. 2A.

First, the valve 33a and the pump 33 are operated, so that the aqueous liquid 21 is introduced to the bath 20 from the inlet 36 as shown in Fig. 3A. Next, as shown in Fig. 3B, a voltage is applied between the ion-adsorbing electrode 11 and the counter electrode 13 so that the ion-adsorbing electrode 11 serves as a cathode. This step is the same as the step shown in Fig. 1B. The aqueous liquid 21 is disinfected in this step as described in Embodiment 1.

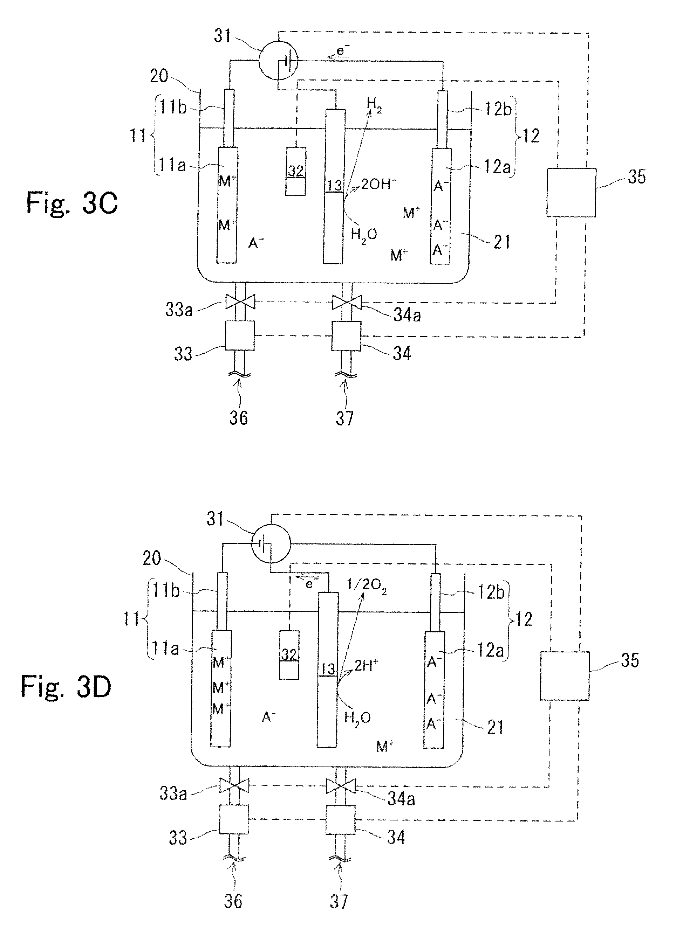

When the controller 35 detects that the pH reaches a specific value less than 5 (e.g., 4 or less), the following step is performed immediately or after a while. Specifically, as shown in Fig. 3C, a voltage is applied between the ion-adsorbing electrode 12 and the counter electrode 13 so that the ion-adsorbing electrode 12 serves as an anode. With this voltage application, the anion A- in the aqueous liquid 21 is adsorbed by the ion-adsorbing electrode 12. Water is electrolyzed at the counter electrode 13, and thus hydroxide ion and hydrogen gas are generated. This voltage application is performed until the pH of the aqueous liquid 21 reaches a specific value greater than 9 (e.g., 10 or more).

The step shown in Fig. 3C provides disinfection with the alkaline aqueous liquid 21, disinfection with the reductive power of the generated hydrogen, and disinfection with the reductive power of the surface of the counter electrode 13.

When the pH is 10 and the hydrogen partial pressure is 1 atm, the reduction potential E₀ = 0.000 - 0.0591pH + 0.0295logP(H₂) = -0.59 volts. When the pH is 12 and the hydrogen partial pressure is 1 atm, the reduction potential E₀ = -0.71 volts. Because the electrode potential of the counter electrodes 13 is polarized due to the hydrogen gas generation, the electrode potential of the counter electrode 13 is lower than the above-mentioned reduction potential. Accordingly, strong reducing power works at the surface of the counter electrode 13, and thus disinfection occurs at the surface of the counter electrode 13, and the aqueous liquid 21 itself is in a state having strong reducing power. Moreover, the decomposition of organic matter, etc. may occur at the surface of the counter electrode 13.

When the controller 35 detects that the pH reaches a specific value, the following step is performed immediately, or after a while. Specifically, as shown in Fig. 3D, a voltage is applied between the ion-adsorbing electrode 11 and the counter electrode 13 so that the ion-adsorbing electrode 11 serves as a cathode. With this voltage application, the reaction shown in Fig. 1B occurs, and thus the pH of aqueous liquid 21 decreases. This voltage application is performed until the pH of the aqueous liquid 21 reaches a specific value in the range of 5 to 9.

Thus the disinfection treatment of the aqueous liquid 21 is completed, and then the valve 34a and the pump 34 are operated to discharge the aqueous liquid 21, which is used as a disinfected liquid, from the outlet 37.

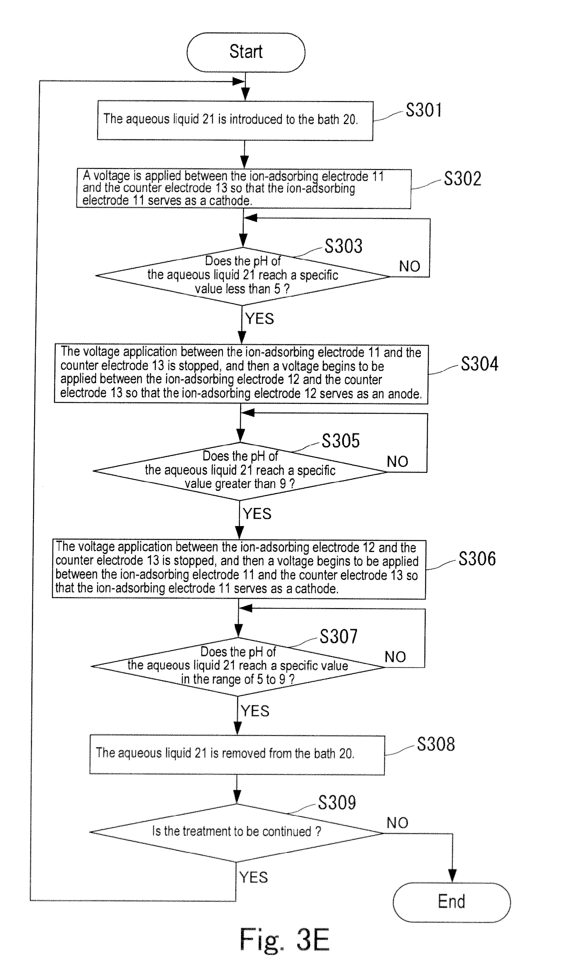

Fig. 3E shows the steps performed in the method of Embodiment 3. First, the valve 33a and the pump 33 are driven, so that the aqueous liquid 21 is introduced to the bath 20 (S301). Next, a voltage begins to be applied between the ion-adsorbing electrode 11 and the counter electrode 13 so that the ion-adsorbing electrode 11 serves as a cathode (S302). This voltage application is continued until the pH of the aqueous liquid 21 reaches a specific value less than 5 (S303). When the pH of the aqueous liquid 21 reaches a specific value less than 5, the voltage application between the ion-adsorbing electrode 11 and the counter electrode 13 is stopped, and then a voltage begins to be applied between the ion-adsorbing electrode 12 and the counter electrode 13 (S304). This voltage application is continued until the pH of the aqueous liquid 21 reaches a specific value greater than 9 (S305). When the pH of the aqueous liquid 21 reaches a specific value greater than 9, the voltage application between the ion-adsorbing electrode 12 and the counter electrode 13 is stopped, and then a voltage begins to be applied between the ion-adsorbing electrodes 11 and the counter electrodes 13 (S306). This voltage application is continued until the pH of the aqueous liquid 21 reaches a specific value in the range of 5 to 9 (S307). After the pH of aqueous liquid 21 reaches a specific value in the range of 5 to 9, the aqueous liquid 21 is removed from the bath 20 to be used. It also is possible to use the aqueous liquid 21 as is present in the bath 20. In the case where the treatment is to be continued, the treatment is continued again from Step S301 (S309).

The memory of the controller of the disinfection device 200 in Embodiment 3 stores the program for performing the above-mentioned treatment. The same steps as part of the steps shown in Fig. 3E also are performed in the device of other Embodiments. Specifically, next step will be performed when the pH of the aqueous liquid reaches a specific value predetermined for each step.

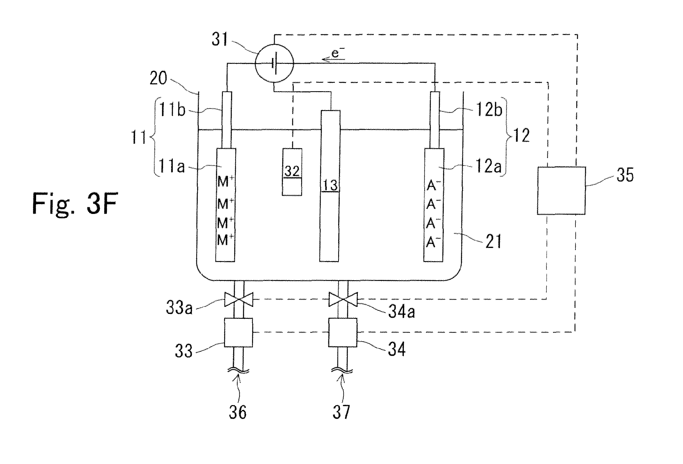

After the step shown in Fig. 3D, a voltage may be applied between the ion-adsorbing electrode 11 and the ion-adsorbing electrode 12 so that the ion-adsorbing electrode 11 serves as a cathode. This voltage application allows the concentration of the cations and anions in the aqueous liquid 21 to decrease as shown in Fig. 3F.

### [Embodiment 4]

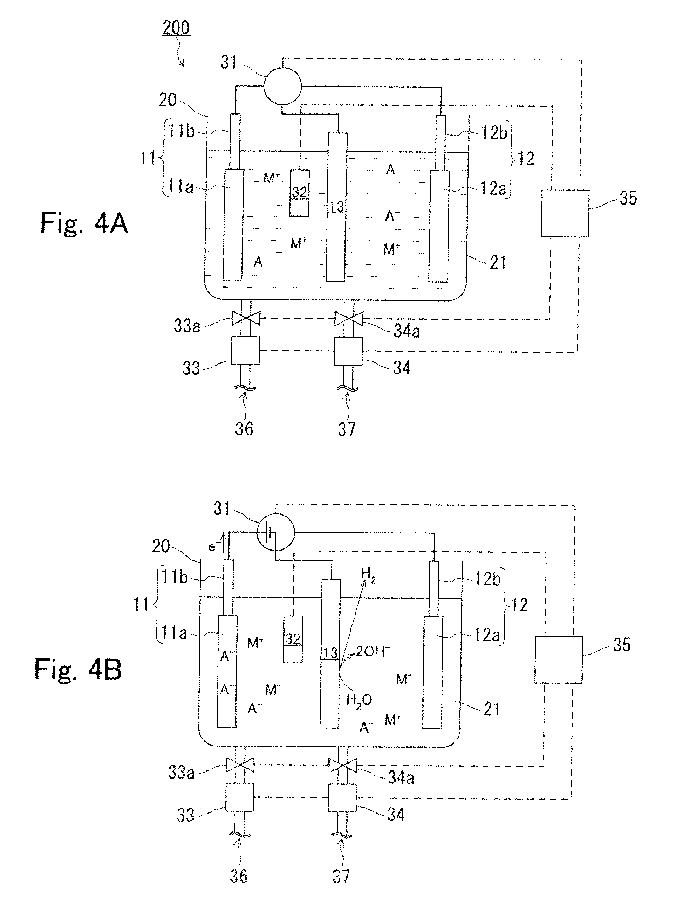

In Embodiment 4, an example is described with regard to the second example of the third method and a device for performing the method. In each step of Embodiment 4, a voltage is applied in the opposite direction to that in Embodiment 3. Fig. 4A shows a disinfection device of Embodiment 4. The disinfection device 200 shown in Fig. 4A has the same structure as the device shown in Fig. 2A.

First, the valve 33a and the pump 33 are operated, so that the aqueous liquid 21 is introduced to the bath 20 from the inlet 36 as shown in Fig. 4A. Next, as shown in Fig. 4B, a voltage is applied between the ion-adsorbing electrode 11 and the counter electrode 13 so that the ion-adsorbing electrode 11 serves as an anode. This voltage application is performed until the pH of the aqueous liquid 21 reaches a specific value greater than 9. In this step, the same reaction as that occurring in the step shown in Fig. 3C occurs. The aqueous liquid 21 is disinfected in this step as described in Embodiment 3.

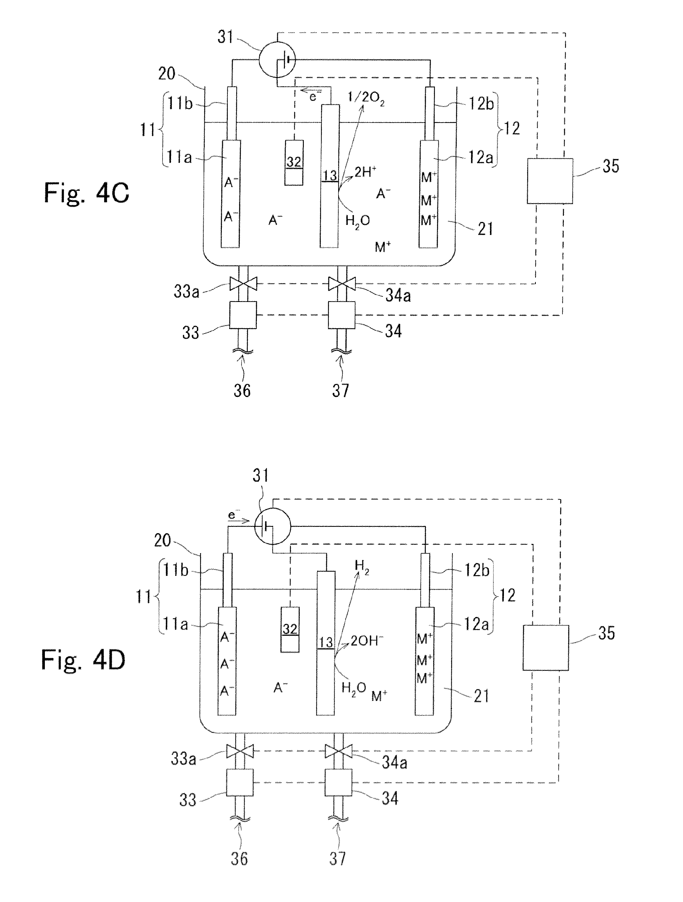

When the controller 35 detects that the pH reaches a specific value, the following step is performed immediately or after a while. Specifically, as shown in Fig. 4C, a voltage is applied between the ion-adsorbing electrode 12 and the counter electrodes 13 so that the ion-adsorbing electrode 12 serves as a cathode. This voltage application is performed until the pH of the aqueous liquid 21 reaches a specific value less then 5. In this step, the same reaction as that occurring in the step shown in Fig. 1B occurs. The aqueous liquid 21 is disinfected in this step as described in Embodiments 1.

When the controller 35 detects that the pH reaches a specific value, the following step is performed immediately or after a while. Specifically, as shown in Fig. 4D, a voltage is applied between the ion-adsorbing electrode 11 and the counter electrode 13 so that the ion-adsorbing electrode 11 serves as an anode. This voltage application is continued until the pH of the aqueous liquid 21 reaches a specific value in the range of 5 to 9.

Thus the disinfection treatment of the aqueous liquid 21 is completed, and then the valve 34a and the pump 34 are operated to discharge the aqueous liquid 21, which is used as a disinfected liquid, from the outlet 37.

### [Embodiment 5]

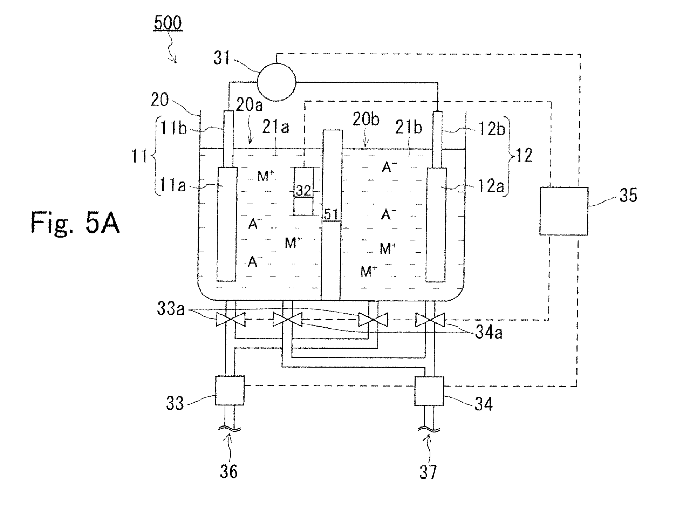

In Embodiment 5, an example is described with regard to the fourth method and a device for performing the method. Fig. 5A shows a disinfection device of Embodiment 5. A disinfection device 500 shown in Fig. 5A is different from the disinfection device 200 shown in Fig. 2A in that the disinfection device 500 includes a counter electrode 51 instead of the counter electrode 13.

The counter electrode 51 functions as a partition that divides the bath 20 into a bath 20a and a bath 20b. The counter electrode 51 is a metal plate and does not allow liquids and ions to pass therethrough. The counter electrode 51 is not connected to the power supply 31, and is in an electrically floating state.

The inlet 36 and the outlet 37 are connected to the bath 20a and the bath 20b, respectively. The first ion-adsorbing electrode 11 is placed in the bath 20a, and the second ion-adsorbing electrode 12 is placed in the bath 20b.

First, the valve 33a and the pump 33 are operated, so that the aqueous liquid 21 is introduced to the bath 20a and 20b from the inlet 36 as shown in Fig. 5A. The aqueous liquid 21 in the bath 20 is divided into the aqueous liquid 21a and the aqueous liquid 21b by the counter electrode 51.

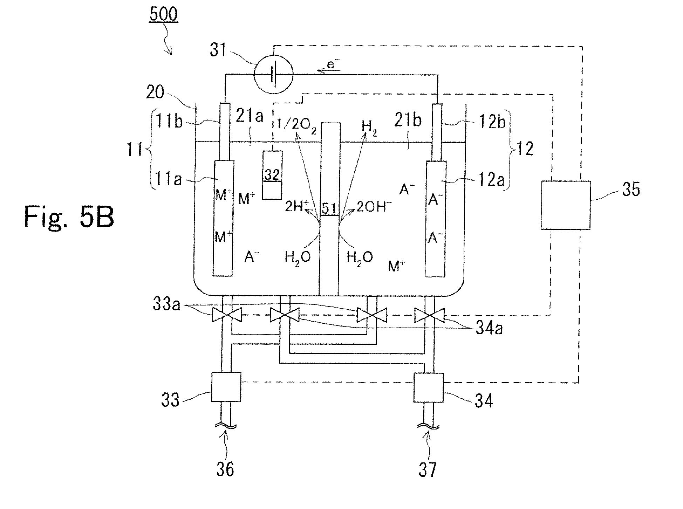

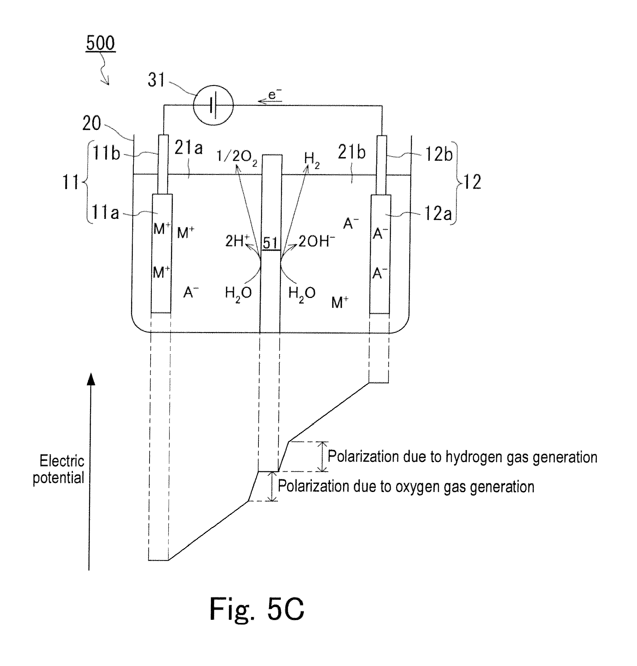

Next, as shown in Fig. 5B, a voltage is applied between the ion-adsorbing electrode 11 and the ion-adsorbing electrode 12 so that the ion-adsorbing electrode 11 serves as a cathode. Fig. 5C schematically shows the electric potential gradient between the ion-adsorbing electrode 11 and the ion-adsorbing electrode 12 at this time. As shown in Fig. 5C, the voltage application between the ion-adsorbing electrode 11 and the ion-adsorbing electrode 12 works as both a voltage application between the ion-adsorbing electrode 11 and the counter electrode 13 and a voltage application between the ion-adsorbing electrode 12 and the counter electrode 13. That is, the same reaction as that shown in Fig. 1B occurs in the bath 20a, and the same reaction as that shown in Fig. 3C occurs in the bath 20b. As a result, the aqueous liquid 21a in the bath 20a and the aqueous liquid 21b in the bath 20b are disinfected. The voltage application is continued until the pH of the aqueous liquid 21a and the pH of the aqueous liquid 21b reach a specific value less than 5 and a specific value greater than 9, respectively.

Next, the valve 34a and the pump 34 are operated, so that the aqueous liquid 21a in the bath 20a and the aqueous liquid 21b in the bath 20b are discharged from the outlet 37 to be mixed. As a result, a neutral aqueous liquid is produced.

As mentioned above, in the disinfection device 500, a voltage may be applied in the opposite direction after the step shown in Fig. 5B to change the pH of the aqueous liquid 21a to a value greater than 9 and to change the pH of the aqueous liquid 21b to a value less than 5. After that, the aqueous liquid 21a and the aqueous liquid 21b may be mixed.

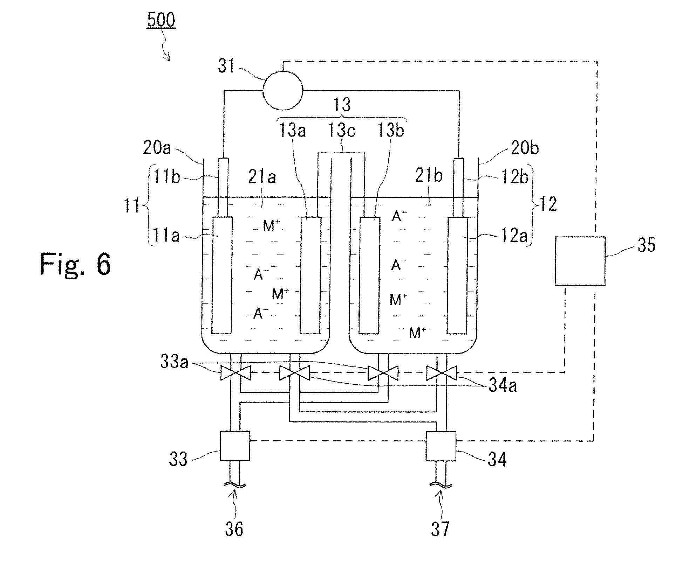

The bath 20a and the bath 20b may be separated as shown in Fig. 6. The counter electrode 13 includes a counter electrode 13a placed in the bath 20a, a counter electrode 13b placed in the bath 20b, and a wiring 13c that connects them. The counter electrode 13 is in an electrically floating state. When a voltage is applied between the ion-adsorbing electrode 11 and the ion-adsorbing electrode 12 so that the ion-adsorbing electrode 11 serves as a cathode, the same reaction as that shown in Fig. 5B occurs.

### [Embodiment 6]

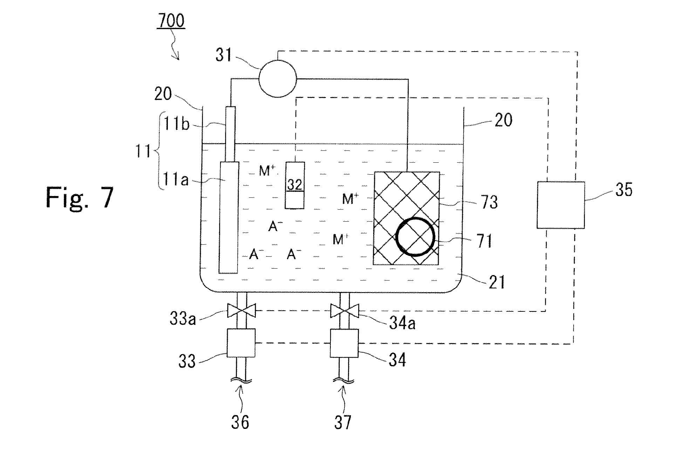

In Embodiment 6, an example is described with regard to a method and a device for disinfecting instruments. Fig. 7 shows a disinfection device of Embodiment 6. A disinfection device 700 shown in Fig. 7 is different from the disinfection device 100 shown in Fig. 1 in that the disinfection device 700 includes a counter electrode 73 instead of the counter electrode 13. The counter electrode 73 is a basket-shaped electrode formed of metal wire. An instrument 71 to be disinfected is placed inside the counter electrode 73. The instruments disinfected with this device preferably have resistance to acids and/or alkalis.

In the disinfection device 700, the same steps as that in Embodiment 1 are performed. When the instrument 71 is electrically conductive, the electric potential of the instrument 71 approaches the electric potential of the counter electrode 73. As a result, the surface of the instrument 71 has strong oxidative power as well as the surface of the counter electrode 73, and thereby the surface of the instrument 71 is disinfected. It should be noted that the structure of Embodiment 6 is applicable to the devices of other Embodiments.

### [Embodiment 7]

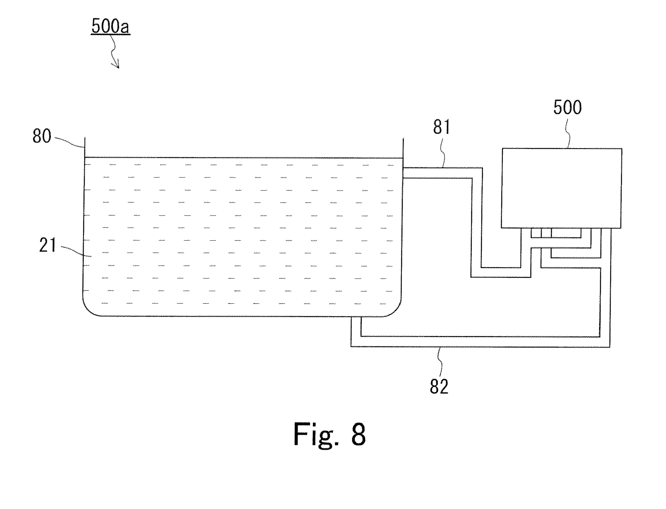

In Embodiment 7, an example is described with regard to a method and a device for disinfecting an aqueous liquid stored in a container. Fig. 8 shown a disinfection device 500a of Embodiment 7.

The disinfection device 500a includes a container 80 and the disinfection device 500 that is connected to the container 80 via pipes 81 and 82. The disinfection device 500 is the disinfection device described in Embodiment 5. One of the pipes 81 and 82 is connected to an inlet of the disinfection device 500, and the other is connected to an outlet of the disinfection device 500. The aqueous liquid 21 is placed in the container 80. The pH sensor 32 of the disinfection device 500 may be placed in the container 80.

The container 80 may be a water container, such as a bathtub and a pool. Alternatively, the container 80 may be a disinfection bath for disinfecting instruments etc. therein. Moreover, the container 80 may be replaced with a circulating water system, such as a cooling tower. In one aspect, the disinfection device 500 shown in Fig. 8 is connected to a system including the aqueous liquid 21.

The disinfection device 500 performs the steps described in Embodiment 5. As a result, the aqueous liquid 21 introduced into the disinfection device 500 from the container 80 is disinfected, and then returned to the container 80. Although the aqueous liquid that can be disinfected at one time is part of the aqueous liquid 21 in the container 80, the increase of the germs in the aqueous liquid 21 can be suppressed by repeating the treatment.

The disinfection device described in Embodiments 1 to 4 mentioned above may be used instead of the disinfection device 500.

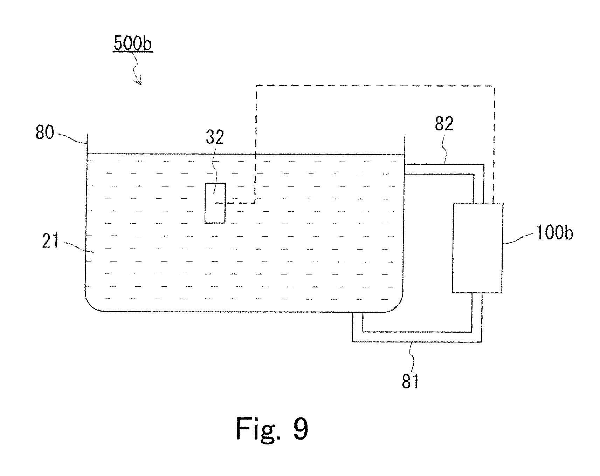

Moreover, the disinfection device described in Embodiments 1 to 7 may perform the treatment as a flow-through process. In one of the preferred devices in that case, the electrodes are placed between the inlet for introducing the aqueous liquid and the outlet for discharging the aqueous liquid. That is, the inlet, the electrodes, and the outlet may be placed so that the ion-adsorbing electrode and the counter electrode are placed in the middle of the flow of the aqueous liquid in the bath (container). For example, the disinfection device performing a flow-through process may be used instead of the disinfection device 500 shown in Fig. 8. Such an embodiment is shown in Fig. 9. A disinfection device 500b shown in Fig. 9 includes the container 80 and the disinfection device 100b that is connected to the container 80 via the pipes 81 and 82. In the disinfection device 500b, the pH sensor 32 is placed in the container 80. A plurality of the disinfection devices 100b may be connected to the container 80 in parallel or in series.

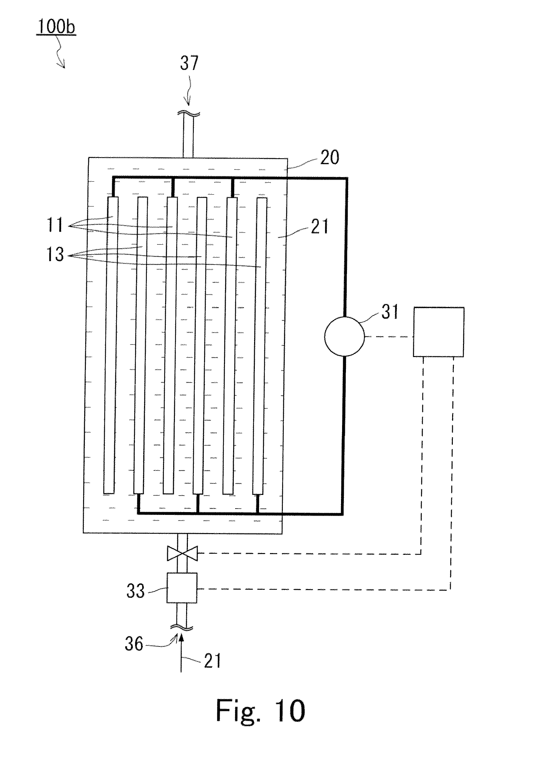

Fig. 10 shows the disinfection device 100b in detail. The disinfection device 100b is different from the device 100 of Embodiment 1 in the shape of the bath 20, in that the device 100b does not include the valve 34a and the pump 34, in that the inlet 36 and the outset 37 are connected to the bath 20, and in that the pH sensor 32 is placed in the container 80. The rest is the same as the device 100 of Embodiment 1. In the disinfection device 100b, the aqueous liquid 21 is continuously introduced from the inlet 36 while the aqueous liquid 21 is continuously discharged from the outlet 37. The internal volume of the bath 20 is smaller than the volume of water present in the container 80. The above-mentioned steps are performed in a state in which the aqueous liquid 21 is moving inside the bath 20. In one aspect, the disinfection device 100b shown in Fig. 9 is connected to a system including the aqueous liquid 21.

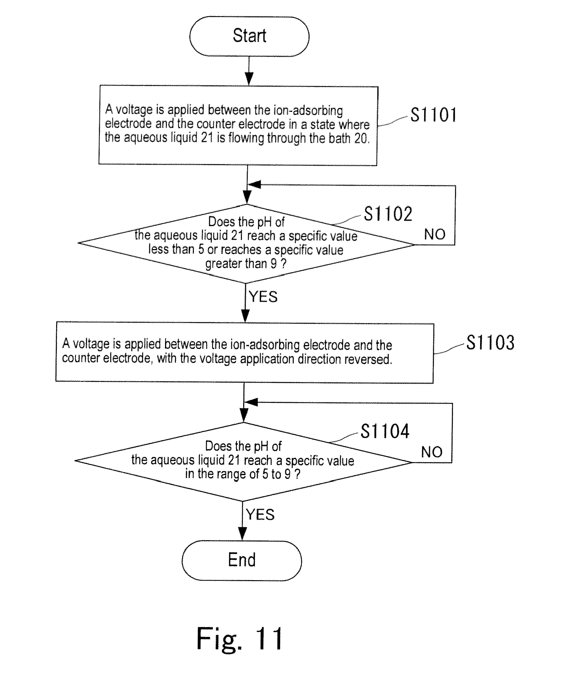

In the disinfection device 500b, the aqueous liquid 21 in the container 80 is introduced into the disinfection device 100b via the pipe 81, and is treated. After that, the aqueous liquid 21 is returned to the container 80 via the pipe 82. By performing the voltage application of step (i), the pH of the aqueous liquid 21 in the container 80 changes gradually. The voltage application in step (i) is performed until the pH of the aqueous liquid 21 reaches a specific value less than 5 or greater than 9. Next, step (ii) mentioned above is performed. In addition to step (ii), other steps mentioned above may be performed. Fig. 11 shows an example of the treatment in the case where only step (i) and step (ii) are performed with the disinfection device 500b.

First, a voltage is applied between the ion-adsorbing electrode and the counter electrode in a state where the aqueous liquid 21 is flowing through the bath 20 of the disinfection device 100b (S1101). This voltage application is continued until the pH of the aqueous liquid 21 reaches a specific value less than 5 or greater than 9 (S1102). When the controller detects that the pH of the aqueous liquid 21 reaches the specific value, a voltage is applied immediately or after a while between the ion-adsorbing electrode and the counter electrode, with the voltage application direction reversed (S1103). This voltage application is continued until the pH of the aqueous liquid 21 reaches a specific value in the range of 5 to 9 (S1104). Thus, the aqueous liquid 21 is disinfected.

The disinfection device 500b shown in Fig. 9 can disinfect a large amount of water using the small disinfection device 100b. In this case, the distance between the electrodes can be small, so that the voltage drop due to the resistance of the aqueous liquid can be small. Accordingly, the voltage applied between the electrodes can be low, enabling the use of inexpensive power supplies. In addition, two disinfection devices may be connected in parallel, and step (ii) may be performed in a way that an aqueous liquid is made acidic using the first disinfection device, another aqueous liquid is made alkaline using the second disinfection device, and then these aqueous liquids are mixed.

### [Example of the ion-adsorbing electrodes]

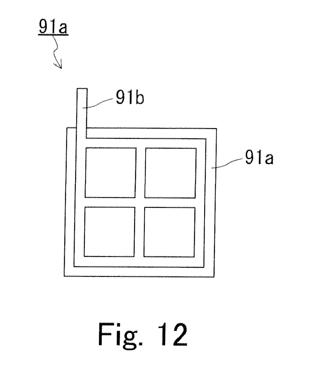

Fig. 12 shows an example of the ion-adsorbing electrodes used in the disinfection device of the present invention. The ion-adsorbing electrode 91 shown in Fig. 12 includes an activated carbon fiber cloth 91a and a collector 91b attached thereto. The collector 91b can reduce the electric potential change in the activated carbon fiber cloth 91a.

### Examples

Examples in which aqueous liquids were disinfected using the method of the present invention are described below. In Examples, a disinfection device including a container, an ion-adsorbing electrode and a counter electrode, both of which are placed in the container, was used. In the following examples, the pH values of test liquids are that measured in advance using dummy test liquids. That is, the pH values of the test liquids are that of the dummy test liquids subjected to voltage application on the same conditions as that in Examples.

### [Example 1]

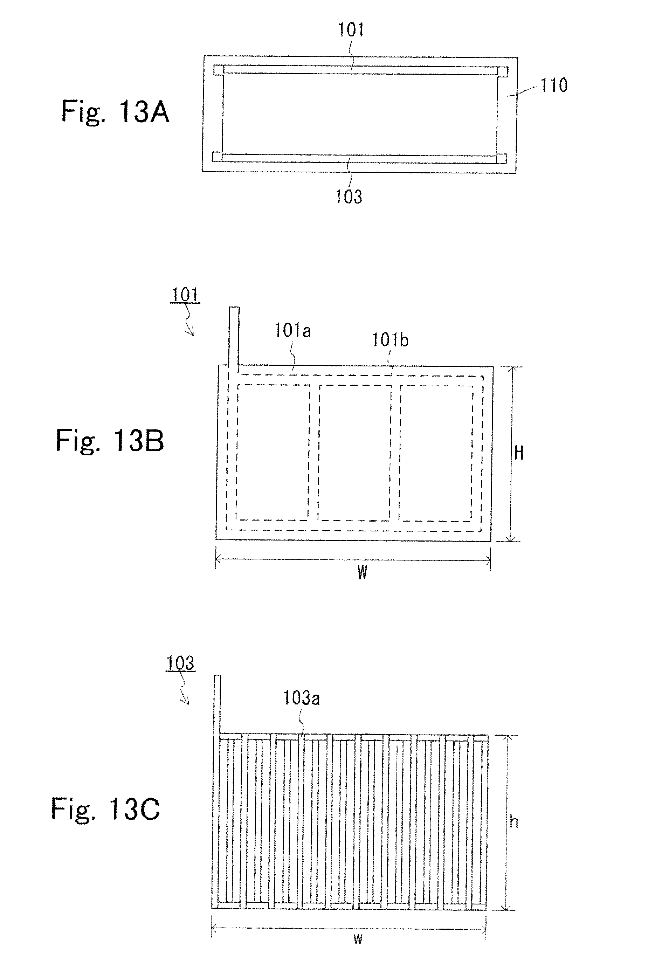

Fig. 13A shows the top view of the disinfection device used herein. The disinfection device shown in Fig. 13A included a container 110, an ion-adsorbing electrode 101 and a counter electrode 103 placed in the container. The container 110 was about 80 mm in height, and the inside dimension thereof was about 20 mm in length and about 90 mm in width. The ion-adsorbing electrode 101 and the counter electrode 103 were placed so that they faced each other with a distance of about 20 mm. The wire that constituted the counter electrode 103 was placed to be parallel to the surface of the ion-adsorbing electrode 101.

Fig. 13B shows a side view of the ion-adsorbing electrode 101. The ion-adsorbing electrode 101 was about 70 mm in height H and about 90 mm in width W. An activated carbon fiber cloth (ACC-5092-10 made by NIPPON KYNOL, Inc., area density: 200 g/m², thickness: 0.53 mm, specific surface area: 1100m²/g) was used as the electrically conductive material of the ion-adsorbing electrode 101. Three activated carbon fiber cloths 101a each having a size of about 70 mm by 90 mm were stacked to be used as the ion-adsorbing electrode 101. The wiring 101b was placed between one of the activated carbon fiber cloths and two of the activated carbon fiber cloths.

Fig. 13C shows a side view of the counter electrode 103. The counter electrode 103 was about 70 mm in height h and about 90 mm in the width w. The counter electrode 103 was formed using titanium wires 103a (about I mm in diameter) coated with platinum. Specifically, twenty of the wires 103a were arranged in stripes, and those ends were connected with the wires 103a to form the counter electrode 103.

First, 120 ml of a test liquid was placed in the disinfection device. A neutral sodium chloride aqueous solution (sodium chloride concentration: 0.78 g/liter) containing germs was used as the test liquid. Next, a voltage was applied between the ion-adsorbing electrode and the counter electrode so that the ion-adsorbing electrode served as an anode. The voltage was applied for 15 minutes so that 200 mA of electric current flowed between the electrodes. This voltage application changed the pH of the test liquid to 13.1. After the voltage application, the voltage application was stopped and the test liquid was allowed to stand for 15 minutes. This standing changed the pH of the test liquid to 12.8. After 15 minutes standing, a voltage began to be applied between the ion-adsorbing electrode and the counter electrode so that the ion-adsorbing electrode served as a cathode. The voltage was applied for 30 minutes so that 200 mA of electric current flowed between the electrodes. This voltage application changed the pH of the test liquid to 2.3. The test liquid was allowed to stand for 15 minutes after the voltage application, thereby the pH was changed to 2.5. The pH of the test liquid 10 minutes after the start of the standing was estimated to be about 2.4 because the pH of the test liquid before the standing was 2.3 and the pH of the test liquid after the standing was 2.5.

After the passage of a predetermined time from the start of the experiment, part of the test liquid was taken out to determine the number of viable cells in it. The test liquid was added to a SCDLP medium (made by NIHON PHARMACEUTICAL CO., LTD.) and cultivated, and then the number of viable cells was determined. As a control experiment, the number of viable cells in a test liquid that has not been disinfected was determined at the start of the test and a predetermined time after the start of the test. The experiment and determination of the number of viable cells were carried out by Japan Food Research Laboratories. The determination of the number of viable cells and the control experiment each were carried out in the same way in the following Examples.

Table 1 shows the relationship between the elapsed time from the start of the test, the pH of the test liquid, and the number of viable cells.

**[Table 1]**

| Germs | Disinfected ? | Number of viable cells (counts/mL) | | | | | | |
|---|---|---|---|---|---|---|---|---|
| | | Initial | 15 min later | 20 min later | 30 min later | 60 min later | 65 min later | 75 min later |
| | | neutral | pH 13.1 | - | pH 12.8 | pH 2.3 | - | pH ab out 2.4 |
| Bacillus subtilis (spore) | Yes | 5.6×10³ | 4.3×10⁵ | 4.3×10⁻⁵ | 5.0×10³ | 5.2×10³ | 5.7×10³ | 4.5×10³ |
| | No | 5.6×10⁵ | - | - | - | - | - | 6.2×10⁵ |
| Escherichia coli | Yes | 8.3×10⁵ | 1.2×10⁵ | 6.4×10⁴ | 2.7×10⁴ | <10 | <10 | <10 |
| | No | 8.3×10³ | - | - | - | - | - | 9.7×10⁵ |
| Staphylococcus aureus | Yes | 4.5×10⁵ | 1.7×10⁵ | 2.0×10⁵ | 3.3×10⁵ | <10 | <10 | <10 |
| | No | 4.5×10⁵ | - | - | - | - | - | 5.1×10⁵ |
| Aspergillus niger | Yes | 5.0×10⁵ | 2.7×10⁴ | 9.1×10³ | 7.8×10³ | 8.4×10³ | 8.6×10³ | 1.0×10⁴ |
| | No | 5.0×10⁵ | - | - | - | - | - | 5.4×10⁵ |
| Cladosporium | Yes | 5.5×10⁵ | 9.8×10² | 2.7×10² | 1.6×10² | <10 | <10 | <10 |
| | No | 5.5×10⁵ | - | - | - | - | - | 3.6×10⁵ |
| Candida | Yes | 4.7×10⁵ | 9.1×10² | 5.2×10² | 4.6×10² | <10 | <10 | <10 |
| | No | 4.7×10⁵ | - | - | - | - | - | 2.9×10⁵ |

As shown in Table 1, although the number of Bacillus subtilis showed little change after alkali treatment, it was decreased to less than 1/100 after acid treatment. The number of Escherichia coli was decreased to less than 1/10 after alkali treatment, and was decreased to less than 1/10,000 after acid treatment. Although the number of Staphylococcus aureus showed little change after alkali treatment, it was decreased to less than 1/10,000 after acid treatment. Although the number ofAspergillus niger was decreased to less than 1/50 after alkali treatment, it showed little change in acid treatment. The number of Cladosporium was decreased to less than 1/1,000 after alkali treatment, and was decreased to less than 1/10,000 after acid treatment. The number of Candida was decreased to less than 1/1,000 after alkali treatment, and was decreased to less than 1/10,000 after acid treatment. As mentioned above, it was demonstrated that the method and of the present invention are capable of disinfecting.

### [Example 2]

In Example 2, an aqueous liquid was disinfected using the same disinfection device as that used in Example 1. However, in Example 2, an aqueous solution of potassium sulfate (K₂SO₄) was used as the aqueous liquid.

First, 120 ml of a test liquid was placed in the disinfection device. A potassium sulfate aqueous solution (potassium sulfate concentration: 1.16 g/liter) containing germs was used as the test liquid. Next, a voltage was applied between the ion-adsorbing electrode and the counter electrode so that the ion-adsorbing electrode served as an anode. The voltage was applied for 15 minutes so that 200 mA of electric current flowed between the electrodes. This voltage application changed the pH of the test liquid to 13.2. After the voltage application, the voltage application was stopped and the test liquid was allowed to stand for 15 minutes. This standing changed the pH of the test liquid to 12.9. After 15 minutes standing, a voltage began to be applied between the ion-adsorbing electrode and the counter electrode so that the ion-adsorbing electrode served as a cathode. The voltage was applied for 30 minutes so that 200 mA of electric current flowed between the electrodes. This voltage application changed the pH of the test liquid to 2.4. After the voltage application, the voltage application was stopped and the test liquid was allowed to stand for 15 minutes. This standing changed the pH of the test liquid to 2.5. Table 2 shows the relationship between the elapsed time from the start of the test, the pH of the test liquid, and the number of viable cells.

**[Table 2]**

| Germs | Disinfected ? | Number of viable cells (counts/mL) | | | | |
|---|---|---|---|---|---|---|
| | | Initial | 15 min later | 30 min later | 60 min later | 75 min later |
| | | neutral | pH 13.2 | pH 12.9 | pH 2.4 | pH 2.5 |
| Bacillus subtilis (spore) | Yes | 7.3×10⁵ | 4.9×10⁵ | 4.1×10⁵ | 3.6×10⁵ | 3.7×10⁵ |
| | No | 7.3×10⁵ | - | - | - | 6.3×10⁵ |
| Escherichia coli | Yes | 8.5×10⁵ | 1.3×10⁵ | 3.1×10⁴ | 9.0×10² | 9.6×10² |
| | No | 8.5×10⁵ | - | - | - | 9.4×10⁵ |
| Staphylococcus aureus | Yes | 7.3×10⁵ | 4.1×10⁵ | 3.5×10⁵ | <10 | <10 |
| | No | 7.3×10⁵ | - | - | - | 9.1×10⁵ |

As shown in Table 2, the disinfection effect was provided in the case where a potassium sulfate aqueous solution was used as the aqueous liquid as well as the case where a sodium chloride aqueous solution was used as the aqueous liquid.

### [Example 3]

In Example 3, an aqueous liquid was disinfected using the same disinfection device as that used in Example 1. However, in Example 3, a commercially available mineral water (whose electric conductivity is 208 µS/cm) was used as the aqueous liquid.

First, 120 ml of a test liquid was placed in the disinfection devise. A mineral water containing germs was used as the test liquid. Next, a voltage was applied between the ion-adsorbing electrode and the counter electrode so that the ion-adsorbing electrode served as an anode. The voltage was applied for 15 minutes so that 200 mA of electric current flowed between the electrodes. This voltage application changed the pH of the test liquid to 10.5. Next, a voltage was applied between the electrodes for 15 minutes so that 20 mA of electric current flowed between the electrodes and so that the ion-adsorbing electrode served as an anode. This voltage application changed the pH of the test liquid to 10.6. Next, a voltage was applied between the electrodes for 30 minutes so that 200 mA of electric current flowed between the electrodes and so that the ion-adsorbing electrode served as a cathode. This voltage application changed the pH of the test liquid to 3.4. Next, a voltage was applied between the electrodes for 15 minutes so that 20 mA of electric current flowed between the electrodes and so that the ion-adsorbing electrode served as a cathode. This voltage application changed the pH of the test liquid to 3.5. Table 3 shows the relationship between the elapsed time from the start of the test, the pH of the test liquid, and the number of viable cells.

**[Table 3]**

| Germs | Disinfected ? | Number of viable cells (counts/mL) | | | | |
|---|---|---|---|---|---|---|
| | | Initial | 15 min later | 30 min later | 60 min later | 75 min later |
| | | neutral | pH 10.5 | pH 10.6 | pH 3.4 | pH 3.5 |
| Bacillus subtilis (spore) | Yes | 6.9×10⁵ | 5.4×10⁴ | 5.4×10⁴ | 7.1×10⁴ | 7.8×10⁴ |
| | No | 6.9×10⁵ | - | - | - | 8.2×10⁵ |
| Escherichia coli | Yes | 9.5×10⁵ | 8.4×10⁴ | 7.5×10⁴ | <10 | <10 |
| | No | 9.5×10⁵ | - | - | - | 3.8×10⁵ |
| Staphylococcus aureus | Yes | 8.4×10⁵ | 1.7×10⁴ | 8.2×10³ | <10 | <10 |
| | No | 8.4×10⁵ | - | - | - | 9.3×10⁵ |

As shown in Table 3, the disinfection effect was provided in a mineral water to which salt was not added. That is, it was demonstrated that the method and device of the present invention are capable of providing disinfection for tap water and river water.

### [Example 4]

In Example 4, an aqueous liquid was disinfected using the same disinfection device as that used in Example 1. However, in Example 4, two sodium chloride aqueous solutions having different concentration were used as the aqueous liquid. Specifically, sodium chloride aqueous solutions having sodium chloride concentration of 0.78g/liter or 1.56 g/liter were used.

First, 120 ml of a test liquid was placed in the disinfection device. A sodium chloride aqueous solution (sodium chloride concentration: 0.78 g/liter) containing Bacillus subtilis was used as the test liquid. Next, a voltage was applied between the ion-adsorbing electrode and the counter electrode so that the ion-adsorbing electrode servad as an anode. The voltage was applied for 30 minutes so that 200 mA of electric current flowed between the electrodes. This voltage application changed the pH of the test liquid to 13.3. Next, a voltage was applied between the electrodes for 5 minutes so that 20 mA of electric current flowed between the electrodes and so that the ion-adsorbing electrode served as an anode. The pH of the test liquid after the voltage application was 13.3. Next, a voltage was applied between the electrodes for 60 minutes so that 200 mA of electric current flowed between the electrodes and so that the ion-adsorbing electrode served as a cathode. This voltage application changed the pH of the test liquid to 2.4. Next, a voltage was applied between the electrodes for 25 minutes so that 20 mA of electric current flowed between the electrodes and so that the ion-adsorbing electrode served as a cathode. The pH of the test liquid after the voltage application was 2.4.

Furthermore, the same examination was carried out using a sodium chloride aqueous solution (sodium chloride concentration: 1.56g/liter) containing Bacillus subtilis. Furthermore, the same examination was carried out using a sodium chloride aqueous solution (sodium chloride concentration: 0.78g/liter) containing Aspergillus niger. However, in the examination using Aspergillus niger, the voltage was applied in a direction opposite to that in the examination using Bacillus subtilis. Tables 4-6 show the relationship between the elapsed time from the start of the test, the pH of the test liquid, and the number of viable cells. As shown in Tables 4 to 6, the disinfection effect was provided also under the conditions of Example 4.

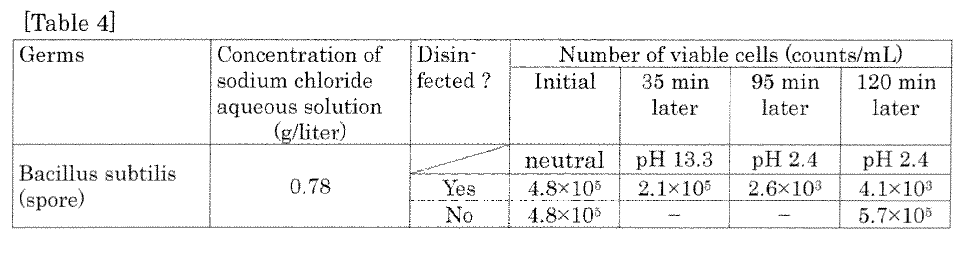

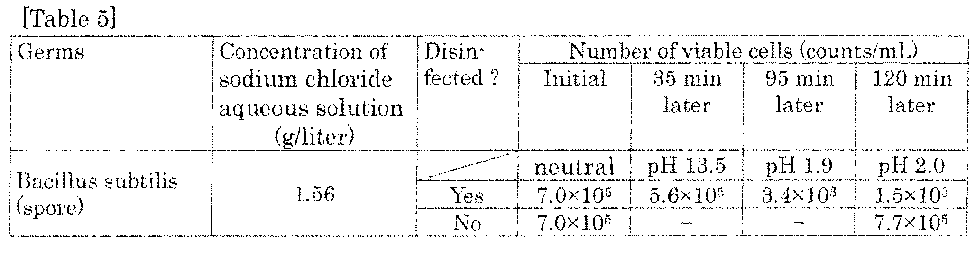

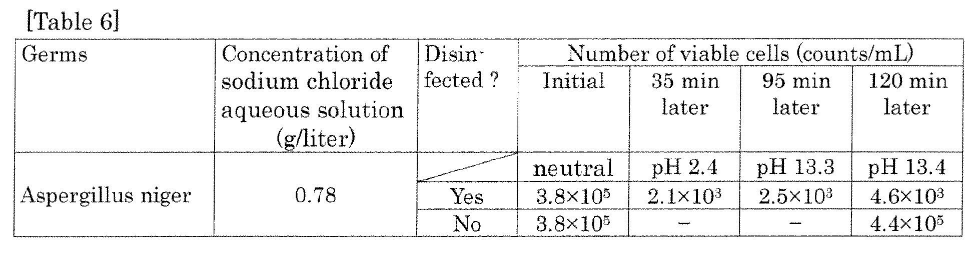

### [Example 5]

In Example 5, the aqueous liquid was disinfected using the same disinfection device as that used in Example 1.

First, 120 ml of a test liquid was placed in the disinfection device. A sodium chloride aqueous solution (sodium chloride concentration: 0.78 g/liter) containing Staphylococcus aureus or Candida was used as the test liquid. Next, a voltage was applied between the ion-adsorbing electrode and the counter electrode so that the ion-adsorbing electrode served as a cathode. The voltage was applied for 7 minutes so that 1.4 mA of electric current flowed between the electrodes. After the voltage application, the voltage application was stopped and the test liquid was allowed to stand for 4 minutes. In the middle of this standing (about 8 minutes after the start of the test), the number of viable cells and the pH were measured. The pH was 4.9.

Next, a voltage was applied between the electrodes for 7 minutes so that the ion-adsorbing electrode served as a cathode and so that 4.6 mA of electric current flowed between the electrodes. After the voltage application, the voltage application was stopped and the test liquid was allowed to stand for 4 minutes. In the middle of this standing (about 19 minutes after the start of the test), the number of viable cells and the pH were measured. The pH was 3.9.

Next, a voltage was applied between the electrodes for 7 minutes so that the ion-adsorbing electrode served as a cathode and so that 37 mA of electric current flowed between the electrodes. After the voltage application, the voltage application stopped and the test liquid was allowed to stand for 4 minutes. In the middle of this standing (about 30 minutes after the start of the test), the number of viable cells and the pH were measured. The pH was 2.9. Table 7 shows the relationship between the elapsed time from the start of the test, the pH of the test liquid, and the number of viable cells. As shown in Table 7, the disinfection effect was provided also under the conditions of Example 5.

**[Table 7]**

| Germs | Disinfected ? | Number of viable cells (counts/mL) | | | |
|---|---|---|---|---|---|
| | | Initial | about 8 min later | about 19 min later | about 30 min later |
| | | neutral | pH 4.9 | pH 3.9 | pH 2.9 |
| Staphylococcus aureus | Yes | 5.3×10⁵ | 2.9×10⁵ | 2.2×10² | <10 |
| | No | 5.3×10⁵ | - | - | - |
| Candida | Yes | 5.9×10⁵ | 5.2×10⁵ | 4.4×10⁵ | 1.8×10² |
| | No | 5.9×10⁵ | - | - | - |

### INDUSTRIAL APPLICABILITY

The present invention can be applied to disinfection methods and disinfection devices. For example, the present invention can be applied to methods and devices for producing drinking water, methods and devices for disinfecting drinking water, methods and devices for disinfecting water in a bath or a swimming pool, and methods and devices for disinfecting instruments. Since the disinfection method and disinfection device of the present invention can be miniaturized, they can be used even in an area of situation with no electric power supply. Accordingly, the disinfection method and disinfection device of the present invention can preferably be used in emergencies such as a disaster.

## Claims

1. A disinfection method comprising the steps of:
(i) applying a voltage, in an aqueous liquid, between a counter electrode and a first ion-adsorbing electrode containing a first electrically conductive material capable of adsorbing an ion reversibly, thereby changing a pH of the aqueous liquid to a value less than 5 or to a value greater than 9; and
(ii) adjusting the pH of the aqueous liquid to a range of 5 to 9, wherein step (i) and step (ii) are performed in this order.

2. The disinfection method according to claim 1, wherein step (i) is performed as a batch process.

3. The disinfection method according to claim 1, wherein the first ion-adsorbing electrode and the counter electrode are placed in a bath connected to a system including the aqueous liquid, and step (i) is performed in a state where the aqueous liquid flows through the bath continuously.

4. The disinfection method according to claim 1, wherein step (ii) is performed by applying a voltage between the first ion-adsorbing electrode and a counter electrode in the aqueous liquid.

5. The disinfection method according to claim 1, wherein step (ii) is performed by applying a voltage between a second ion-adsorbing electrode and a counter electrode in the aqueous liquid, and the second ion-adsorbing electrode contains a second electrically conductive material capable of adsorbing an ion reversibly.

6. The disinfection method according to clam 1, further comprising, between step (i) and step (ii), a step (x) in which the pH of the liquid that has been subjected to step (i) is less than 5, the pH of the aqueous liquid is changed to a value greater than 9, and when the pH of the aqueous liquid that has been subjected to step (i) is greater than 9, the pH of the aqueous liquid is changed to a value less than 5.

7. The disinfection method according to clam 1, wherein an object to be disinfected is immersed in the aqueous liquid while the voltage is applied in step (i), thereby disinfecting the object.

8. A disinfection device comprising a first ion-adsorbing electrode, a counter electrode, and a power supply for applying a voltage between the first ion-adsorbing electrode and the counter electrode, wherein
the first ion-adsorbing electrode contains a first electrically conductive material capable of adsorbing an ion reversibly, and the disinfection device performs the steps of:
(i) applying a voltage between the first ion-adsorbing electrode and the counter electrode in an aqueous liquid, thereby changing a pH of the aqueous liquid to a value less than 5 or to a value greater than 9; and
(ii) adjusting the pH of the aqueous liquid to a range of 5 to 9, wherein step (i) and step (ii) are performed in this order.

9. The disinfection device according to claim 8, further comprising a bath in which the aqueous liquid is placed.

10. The disinfection device according to claim 8, further comprising a pH sensor for monitoring the pH of the aqueous liquid.

11. The disinfection device according to claim 8, wherein step (i) is performed as a batch process.

12. The disinfection device according to claim 8, wherein an object to be disinfected is immersed in the aqueous liquid while the voltage is applied in step (i), thereby disinfecting the object.

13. The disinfection device according to claim 8, wherein the disinfection device is connected to a system including the aqueous liquid.

14. The disinfection device according to claim 13, wherein (i) is performed as a flow-through process.

15. The disinfection device according to clam 8, wherein the first electrically conductive material contains activated carbon.
